(19) Europäisches Patentamt
European Patent Office
Office européen des brevets

(11) Veröffentlichungsnummer : **0 626 178 A1**

(12) # EUROPÄISCHE PATENTANMELDUNG

(21) Anmeldenummer : **94810274.4**

(51) Int. Cl.$^5$ : **A61K 37/64**

(22) Anmeldetag : **09.05.94**

(30) Priorität : **17.05.93 CH 1492/93**

(43) Veröffentlichungstag der Anmeldung :
**30.11.94 Patentblatt 94/48**

(84) Benannte Vertragsstaaten :
**AT BE CH DE DK ES FR GB GR IE IT LI LU NL PT SE**

(71) Anmelder : **CIBA-GEIGY AG**
**Klybeckstrasse 141**
**CH-4002 Basel (CH)**

(72) Erfinder : **Rösel, Johannes, Dr.**
**Grenzacherweg 78**
**CH-4125 Riehen (CH)**
Erfinder : **Regenass, Urs Dr.**
**Mühlerain 14**
**CH 4107 Ettingen (CH)**
Erfinder : **Lang, Marc, Dr.**
**Rue de Valdoie 24**
**F-68200 Mulhouse (FR)**
Erfinder : **Bold, Guido, Dr.**
**Bleumatthöhe 16**
**CH-5264 Gipf-Oberfrick (CH)**
Erfinder : **Cumin, Frédéric, Dr.**
**Rue des Prés Bat. 5C/1**
**F-68220 Hagental-Le-Bas (FR)**

(54) **Verwendung von Hemmstoffen von HIV-Aspartatproteasen zur Bekämpfung von Tumorerkrankungen.**

(57) Die vorliegende Erfindung betrifft die Verwendung von Hemmstoffen von HIV-Aspartatproteasen, wie der HIV-1- oder HIV-2-Protease, und ihren Salzen sowie Prodrugs zur Hemmung des Wachstums von Tumoren, insbesondere solchen Tumoren, die nicht auf die Hemmung von HIV-Aspartatproteasen selbst ansprechen, und zur Herstellung von pharmazeutischen Präparaten, die für die Anwendung zur Prophylaxe und insbesondere Therapie von Tumorerkrankungen bestimmt sind, sowie Methoden und pharmazeutische Präparate zur Tumorbehandlung mit diesen Verbindungen.

EP 0 626 178 A1

Die vorliegende Erfindung betrifft die Verwendung von als Hemmstoffe von HIV-Aspartatproteasen, wie der HIV-1- oder HIV-2-Protease, beschriebenen Verbindungen und ihren Salzen, insbesondere den pharmazeutisch verwendbaren Salzen, zur Behandlung von Tumorerkrankungen und zur Herstellung von pharmazeutischen Präparaten, die für die Anwendung zur Prophylaxe und insbesondere Therapie von Tumorerkrankungen bestimmt sind, sowie Methoden zur Tumorbehandlung mit diesen Verbindungen und entsprechende pharmazeutische Präparate.

Die oben genannten Hemmstoffe von HIV-Aspartatproteasen, wie der HIV-1- oder der HIV-2-Protease aus HIV-1 oder HIV-2, die als Erreger von AIDS gelten und beschrieben sind, deren Salze und ihre Herstellung sind insbesondere solche, die in der europäischen Patentanmeldung mit der Anmeldenummer 92810678.0 und der Publikationsnummer EP 0 532 466, die auf den Namen der Anmelderin der vorliegenden Erfindung lautet und am 17. März 1993 veröffentlicht wurde, in der europäischen Patentanmeldung mit der Anmeldenummer 89303539.4 und der Publikationsnummer EP 0 337 714, die auf den Namen der Merck & Co., Inc., New Jersey, lautet und am 18.10.1989 veröffentlicht wurde, in der europäischen Patentanmeldung mit der Anmeldenummer 89308555.5 und der Publikationsnummer EP 0 356 223, die auf den Namen der Merck & Co., Inc., New Jersey, lautet und am 28.02.1990 veröffentlicht wurde, in der europäischen Patentanmeldung mit der Anmeldenummer 90313848.5 und der Publikationsnummer EP 0 434 365, die auf den Namen der Merck & Co., Inc., New Jersey, lautet und am 26.06.1991 veröffentlicht wurde, in der europäischen Patentanmeldung mit der Anmeldenummer 91108828.4 und der Publikationsnummer EP 0 459 465, die auf den Namen der Anmelderin der vorliegenden Erfindung lautet und am 04. Dezember 1991 veröffentlicht wurde, oder in der europäischen Patentanmeldung mit der Anmeldenummer 89810923.6 und der Publikationsnummer EP 0 374 098, die auf den Namen der Anmelderin der vorliegenden Erfindung lautet und am 20. Juni 1990 veröffentlicht wurde, beschrieben sind. In keiner dieser Anmeldungen ist die erfindungsgemässe Verwendung nahegelegt. Englischsprachige Äquivalente der beiden letztgenannten Anmeldungen sind die entsprechenden südafrikanischen Patente ZA 91/4136 (erteilt am 26. Februar 1992) und ZA 89/9558 (erteilt am 29. August 1990). Die genannten Patentanmeldungen werden hiermit durch Bezugnahme inkorporiert; besonders die darin genannten Einzelverbindungen gelten als durch Bezugnahme zum erfindungsgemässen Einsatz als in die hier vorliegende Beschreibung aufgenommen. Auch an Hydroxygruppen veresterte Verbindungen, beispielsweise verestert durch Niederalkanoyl, wie Acetyl, oder insbesondere durch Niederalkoxyniederalkoxyniederalkanoyl oder Niederalkoxyniederalkoxyniederalkoxyniederalkanoyl (herstellbar durch Umsetzung der freien Verbindungen mit aktivierten oder in situ aktivierten Niederalkoxyniederalkoxyniederalkansäuren oder Niederalkoxyniederalkoxyniederalkoxyniederalkansäuren, wie den Säurechloriden, beispielsweise herstellbar in Methylenchlorid mit 1-Chlor-N,N,2-trimethyl-1-propenamin bei etwa 0°C, und anschliessende Umsetzung der Säurechloride (beispielsweise nach Isolierung durch Eindampfen) in Dioxan mit dem jeweiligen Hemmstoff von HIV-Aspartatprotease mit freier Hydroxygruppe in Gegenwart von Pyridin) sind möglich.

Vorzugsweise handelt es sich um Verbindungen der Formel

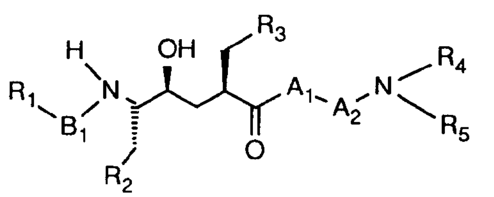

(I),

worin $R_1$ Wasserstoff, Niederalkoxycarbonyl, Heterocyclylcarbonyl, Benzyloxycarbonyl, welches unsubstituiert oder durch bis zu drei unabhängig voneinander aus Fluor, Haloniederalkyl, Niederalkanoyl, Sulfo, Niederalkylsulfonyl und Cyano ausgewählte Reste substituiert ist, Heterocyclyloxycarbonyl, worin Heterocyclyl über ein Kohlenstoffatom gebunden ist, einen der genannten Carbonylreste, worin die bindende Carbonylgruppe durch eine Thiocarbonylgruppe ersetzt ist, Heterocyclylsulfonyl, Niederalkylsulfonyl oder N-(Heterocyclylniederalkyl)-N-niederalkyl-aminocarbonyl bedeutet, $B_1$ eine Bindung oder ein bivalentes Radikal einer $\alpha$-Aminosäure bedeutet, welches N-terminal mit $R_1$ und C-terminal mit der Aminogruppe am $R_2$-$CH_2$- tragenden Kohlenstoffatom verbunden ist, $R_2$ und $R_3$ unabhängig voneinander Phenyl oder Cyclohexyl, wobei diese Reste unsubstituiert oder durch ein bis drei unabhängig aus Hydroxy, Niederalkoxy, Halo, Haloniederalkyl, Sulfo, Niederalkylsulfonyl, Cyano und Nitro ausgewählte Reste substituiert sind, bedeuten, $A_1$ eine Bindung zwischen -C=O und $A_2$ oder ein bivalentes Radikal einer $\alpha$-Aminosäure bedeutet, welches N-terminal mit der Gruppe -C=O und C-terminal mit $A_2$ verbunden ist, $A_2$ ein bivalentes Radikal einer $\alpha$-Aminosäure bedeutet, welches N-terminal mit $A_1$ und C-terminal mit der Gruppe $NR_4R_5$ verbunden ist, oder $A_1$ und $A_2$ zusammen ein bivalentes Radikal eines Dipeptides bilden, dessen zentrale Amidbindung reduziert ist und das N-terminal mit der Gruppe

-C=O und C-terminal mit der Gruppe $NR_4R_5$ verbunden ist, und $R_4$ und $R_5$ gemeinsam mit dem bindenden Stickstoffatom unsubstituiertes oder substituiertes Thiomorpholino oder Morpholino bedeuten, oder Salze dieser Verbindungen, sofern salzbildende Gruppen vorliegen, oder hydroxygeschützte Derivate dieser Verbindungen oder deren Salze, die, zusammen mit ihrer Herstellungsweise, in der europäischen Patentanmeldung mit der Anmeldenummer 92810678.0 und der Publikationsnummer EP 0 532 466, die auf den Namen der Anmelderin der vorliegenden Erfindung lautet und am 17. März 1993 veröffentlicht wurde, beschrieben sind.

Die erfindungsgemäss verwendeten Verbindungen und ihre Salze sind bekannt als Wirkstoffe zur Bekämpfung von durch HIV verursachten Erkrankungen, wie AIDS. Die Wirkungsweise besteht in der Hemmung einer HIV-Aspartatprotease, welche das jeweilige Retrovirus selbst in seinem Genom kodiert und welche notwendig ist für die Reifung kompletter neuer Virionen.

Da Tumorerkrankungen eine der Haupttodesursachen in der heutigen Welt sind, ist es ein ständiges Ziel, Mittel und Wege zur Tumorbehandlung zur Verfügung zu stellen. Insbesondere werden aufgrund der Vielzahl und Verschiedenheit möglicher Tumorerkrankungen ständig neue pharmazeutische Präparate benötigt, die aufgrund ihrer Wirkstoffe zur Behandlung von möglichst vielen oder auch sehr speziellen Tumoren geeignet sind.

Erfindungsgemäss wurde nun überraschend gefunden, dass die oben und unten genannten Hemmstoffe von HIV-Aspartatproteasen eine ausgeprägte Wirksamkeit zur Vorbeugung gegen und Behandlung von Tumorerkrankungen einschliesslich der Bildung von Metastasen besitzen, wie sich etwa aus Tierversuchen, die im Beispielteil illustrativ exemplifiziert sind, ergibt. In diesen Tierversuchen werden Warmblüter, wie Ratten, Meerschweinchen oder insbesondere Mäuse, die an einer Tumorerkrankung leiden oder durch Applikation von Tumorzellen (beispielsweise durch Transplantation von Tumorstücken oder Injektion von Tumorzellinien) eine Tumorerkrankung erleiden, beobachtet, die vor, gleichzeitig oder nach dem Beginn einer Tumorerkrankung mit einem der oben und unten genannten Hemmstoffe von HIV-Aspartatproteasen, insbesondere der Formel I, oder einem Salz davon behandelt werden. Die genannten Hemmstoffe von HIV-Aspartatproteasen hemmen das Wachstum von Tumoren. Sie sind wertvolle Wirkstoffe gegen Tumorerkrankungen.

Bemerkenswert ist die gute Wirksamkeit der oben und unten genannten Hemmstoffe von HIV-Aspartatproteasen gegen Tumorerkrankungen, vor allem solche mit invasivem Tumorwachstum und Metastasenbildung, insbesondere Pancreas-, Lungen-, Darm-, Ovarial- oder Brusttumoren,.

Die oben und unten genannten Hemmstoffe von HIV-Aspartatproteasen, insbesondere der Formel I, können insbesondere zur Prophylaxe und Therapie von Tumorerkrankungen, beispielsweise auch der Bildung von Tumormetastasen, verwendet werden, denen cytokin- oder hormonabhängige Tumorarten, wie Pancreas-, Lungen-, Darm-, Ovarial- oder Brusttumoren, zugrunde liegen.

In erster Linie verwendet man die oben genannten Hemmstoffe von HIV-Aspartatproteasen, insbesondere der Formel I, im Falle von östrogenabhängigen Tumorerkrankungen, beispielsweise Pancreas-, Darm- oder Brusttumoren, die östrogenabhängiges Wachstum zeigen, sowie deren Metastasen.

In allererster Linie werden die oben genannten Hemmstoffe von HIV-Aspartatproteasen, insbesondere der Formel I, im Falle von östrogenabhängigen Brusttumoren verwendet, sowie von deren Metastasen, beispielsweise in axillären Lymphknoten.

Vorzugsweise handelt es sich bei den genannten zu behandelnden Tumorerkrankungen um Erkrankungen an Tumoren, die nicht auf die Hemmung von HIV-Aspartatproteasen (z. B. HIV-1- und/oder HIV-2-Protease) selbst ansprechen.

Beispiele für die Hemmung von Aspartatproteasen und entsprechende Testmethoden finden sich in Jupp et al., Biochem. J. 265, 871 - 878 (1990), die Hemmung der HIV-1-Protease kann beispielsweise nach der in EP 0 532 466 (s. oben) beschriebenen Methode erfolgen.

Die Hemmstoffe von HIV-Aspartatproteasen, insbesondere der Formel I, können als solche oder in Form ihrer pharmazeutischen Präparate verwendet werden, indem man sie enteral oder parenteral zusammen mit geeigneten Hilfs- oder Trägerstoffen bei Warmblütern, insbesondere Menschen, appliziert. Vorzugsweise appliziert man sie auf die Schleimhaut, z.B. intranasal, rektal, vaginal oder auf die Bindehaut des Auges, oder oral, oder auf anderen Wegen, z.B. subkutan, intravenös, intramuskulär, intraperitoneal, oder durch Applikation auf die normale Haut.

Die Dosierung des Wirkstoffes hängt u.a. von der Warmblüterspezies, der Abwehrlage des Organismus, der Applikationsweise und der Art und Lokalisation der Tumorerkrankung ab.

Man verwendet die Hemmstoffe von Aspartatproteasen, insbesondere der Formel I, erfindungsgemäss zur Herstellung von pharmazeutischen Präparaten, die eine pharmakologisch (insbesondere zur Behandlung von Tumorerkrankungen bei Warmblütern, vor allem Menschen, die einer derartigen Behandlung bedürfen) wirksame Menge der Aktivsubstanz zusammen mit signifikanten Mengen von pharmazeutisch verwendbaren Trägermaterialien (mindestens einem) enthalten, die sich zur enteralen, z.B. oralen, oder parenteralen Verabreichung eignen und anorganisch oder organisch, fest oder flüssig sind.

Bei den erfindungsgemäss herstellbaren pharmazeutischen Präparaten handelt es sich um solche zur enteralen, wie nasalen, bukkalen, rektalen oder oralen, oder parenteralen, wie intramuskulären oder intravenösen, Verabreichung an Warmblüter (Menschen und Tiere), welche eine effektive Dosis des pharmakologischen Wirkstoffs allein oder zusammen mit einer signifikanten Menge eines pharmazeutisch anwendbaren Trägermaterials enthalten. Die Dosierung des Wirkstoffs hängt von der Warmblüter-Spezies, dem Körpergewicht, dem Alter und dem individuellen Zustand, individuellen pharmakokinetischen Gegebenheiten, der zu behandelnden Krankheit sowie der Applikationsweise ab.

Die Erfindung betrifft auch pharmazeutische Präparate zur Anwendung in einem Verfahren zur therapeutischen Behandlung des menschlichen oder tierischen Körpers, ein Verfahren zu deren Herstellung (insbesondere als Mittel zur Tumorbehandlung) und eine Methode zur Behandlung von Tumorerkrankungen, insbesondere den oben genannten.

Im Rahmen des Verfahrens zur Tumorbehandlung bei Warmblütern, die an einer Tumorerkrankung leiden, verabreicht man insbesondere Warmblütern, die einer solchen Behandlung bedürfen, eine gegen die Tumorerkrankung wirksame Menge eines Hemmstoffes von HIV-Aspartatproteasen, insbesondere einer Verbindung der Formel I, oder eines pharmazeutisch verwendbaren Salzes davon. Bei der Methode zur Behandlung von Tumorerkrankungen bei Warmblütern verabreicht man insbesondere Warmblütern, die einer solchen Behandlung bedürfen, eine gegen Tumorerkrankungen wirksame Menge eines Hemmstoffes von HIV-Aspartatproteasen, oder eines pharmazeutisch verwendbaren Salzes davon, sofern salzbildende Gruppen vorliegen.

Die an Warmblüter, z. B. Menschen von etwa 70 kg Körpergewicht, zu verabreichenden Dosismengen variieren je nach Spezies, Alter, individuellem Zustand, Applikationsweise und dem jeweiligen Krankheitsbild und liegen insbesondere zwischen etwa 3 mg und etwa 10 g, vorzugsweise zwischen etwa 40 mg und etwa 4 g, z. B. bei ungefähr 150 mg bis 1,5 g pro Person und Tag, verteilt auf vorzugsweise 1 bis 3 Einzeldosen, die z. B. gleich gross sein können. Ueblicherweise erhalten Kinder die halbe Dosis von Erwachsenen. Bei Bedarf kann man die Behandlung solange durchführen, wie es zur Tumorbehandlung und/oder zur Verhinderung der Bildung von Metastasen erforderlich ist.

Die Hemmstoffe von HIV-Aspartatproteasen, insbesondere der Formel I, können allein oder in Kombination mit anderen pharmakologisch wirksamen Substanzen angewendet werden. Zu denken ist z.B. an eine Kombination mit (a) Inhibitoren von Enzymen der Polyaminbiosynthese, z.B. Ornithindecarboxylasehemmern oder S-Adenosylmethionindecarboxylasehemmern, (b) Inhibitoren der Proteinkinase C, (c) Inhibitoren der Protein-Tyrosinkinase, (d) Cytokinen, (e) negativen Wachtumsregulatoren, (f) Aromatasehemmern, (g) Antiöstrogenen oder (h) klassischen zytostatischen Wirkstoffen, wie Adriamycin.

Die pharmazeutischen Präparate enthalten von etwa 1 % bis etwa 95 %, vorzugsweise von etwa 20 % bis etwa 90 % des Wirkstoffes. Erfindungsgemässe hergestellte pharmazeutische Präparate können z. B. in Dosiseinheitsform, wie Ampullen, Vials, Suppositorien, Dragées, Tabletten oder Kapseln, vorliegen. Weitere Applikationsformen sind z.B. Salben, Crèmes, Pasten, Schäume, Tinkturen, Lippenstifte, Tropfen, Sprays, Dispersionen etc.

Die pharmazeutischen Präparate werden erfindungsgemäss in an sich bekannter Weise, z. B. mittels konventioneller Lösungs-, Lyophilisierungs-, Misch-, Granulier- oder Dragierverfahren, hergestellt.

Vorzugsweise verwendet man Lösungen des Wirkstoffes, daneben auch Suspensionen oder Dispersionen, und zwar insbesondere isotonische wässrige Lösungen, Dispersionen oder Suspensionen, wobei diese z. B. bei lyophilisierten Präparaten, welche die Wirksubstanz allein oder zusammen mit einem Trägermaterial, z. B. Mannit, enthalten, vor Gebrauch hergestellt werden können. Die pharmazeutischen Präparate können sterilisiert sein und/oder Hilfsstoffe, z. B. Konservier-, Stabilisier-, Netz- und/oder Emulgiermittel, Löslichkeitsvermittler, Salze zur Regulierung des osmotischen Druckes und/oder Puffer enthalten und werden in an sich bekannter Weise, z. B. mittels konventioneller Lösungs- oder Lyophilisierungsverfahren, hergestellt. Die genannten Lösungen oder Suspensionen können viskositätserhöhende Stoffe, wie Natriumcarboxymethylcellulose, Carboxymethylcellulose, Dextran, Polyvinylpyrrolidon oder Gelatine, enthalten.

Suspensionen in Öl enthalten als ölige Komponente die für Injektionszwecke üblichen vegetabilen, synthetischen oder halbsynthetischen Öle. Als solche sind insbesondere flüssige Fettsäureester zu nennen, die als Säurekomponente eine langkettige Fettsäure mit 8-22, insbesondere 12-22, Kohlenstoffatomen, wie z. B. Laurinsäure, Tridecylsäure, Myristinsäure, Pentadecylsäure, Palmitinsäure, Margarinsäure, Stearinsäure, Arachidinsäure, Behensäure oder entsprechende ungesättigte Säuren, wie z. B. Ölsäure, Elaidinsäure, Erucasäure, Brasidinsäure oder Linolsäure, enthalten, gegebenenfalls unter Zusatz von Antioxidantien, wie z. B. Vitamin E,β-Carotin oder 3,5-Di-tert-butyl-4-hydroxytoluol. Die Alkoholkomponente dieser Fettsäureester hat maximal 6 Kohlenstoffatome und ist ein ein- oder mehrwertiger, z. B. ein-, zwei- oder dreiwertiger Alkohol, z. B. Methanol, Ethanol, Propanol, Butanol oder Pentanol oder deren Isomere, vor allem aber Glycol und Glycerin. Als Fettsäureester sind daher beispielsweise zu nennen: Ethyloleat, Isopropylmyristat, Isopropylpalmitat, "Labrafil M 2375" (Polyoxyethylenglycerintrioleat der Firma Gattefossé, Paris), "Miglyol 812" (Triglycerid

gesättigter Fettsäuren der Kettenlänge $C_8$ bis $C_{12}$ der Firma Hüls AG, Deutschland), besonders aber vegetabile Öle wie Baumwollsaatöl, Mandelöl, Olivenöl, Ricinusöl, Sesamöl, Sojabohnenöl und vor allem Erdnussöl.

Die Herstellung der Injektionspräparate erfolgt in üblicher Weise unter sterilen Bedingungen, ebenso das Abfüllen beispielsweise in Ampullen oder Vialen sowie das Verschliessen der Behälter.

Pharmazeutische Präparate zur oralen Anwendung können erhalten werden, indem man den Wirkstoff mit festen Trägerstoffen kombiniert, ein erhaltenes Gemisch gegebenenfalls granuliert und, wenn erwünscht oder notwendig, nach Zugabe von geeigneten Hilfsstoffen, zu Tabletten, Dragée-Kernen oder Kapseln verarbeitet, oder durch Herstellung von Dispersionen, vorzugsweise mit Phospholipiden, die in Gläschen abgefüllt werden. Dabei kann man die Wirkstoffe auch in Kunststoffträger einbauen, die sie dosiert abgeben oder diffundieren lassen.

Geeignete Trägerstoffe sind insbesondere Füllstoffe, wie Zucker, z. B. Lactose, Dextrose, Sucrose, Saccharose, Glycerin, Mannit oder Sorbit, Cellulosepräparate, und/oder Calciumphosphate, z. B. Tricalciumphosphat oder Calciumhydrogenphosphat, ferner Bindemittel, wie Magnesiumaluminiumsilikat, Stärkekleister unter Verwendung z. B. von Mais-, Weizen-, Reis- oder Kartoffelstärke, Gelatine, Traganth, Methylcellulose, Hydroxypropylmethylcellulose, Natriumcarboxymethylcellulose und/oder Polyvinylpyrrolidon, und/oder, wenn erwünscht, Sprengmittel, wie die oben genannten Stärken, ferner Carboxymethylstärke, quervernetztes Polyvinylpyrrolidon, Agar, Alginsäure oder ein Salz davon, wie Natriumalginat. Hilfsmittel sind in erster Linie Fliessregulier- und Schmiermittel, z. B. Kieselsäure, Talk, Stearinsäure oder Salze davon, wie Magnesium- oder Calciumstearat, und/oder Polyethylenglycol. Dragée-Kerne werden mit geeigneten, gegebenenfalls magensaftresistenten Überzügen versehen, wobei man u.a. konzentrierte Zuckerlösungen, welche gegebenenfalls arabischen Gummi, Talk, Polyvinylpyrrolidon, Polyethylenglycol und/oder Titandioxid enthalten, Lacklösungen in geeigneten organischen Lösungsmitteln oder, zur Herstellung von magensaftresistenten Überzügen, Lösungen von geeigneten Cellulosepräparaten, wie Ethylcellulosephthalat oder Hydroxypropylmethylcellulosephthalat, verwendet. Kapseln sind Steckkapseln aus Gelatine sowie weiche, geschlossene Kapseln aus Gelatine und einem Weichmacher, wie Glycerin oder Sorbit. Die Steckkapseln können den Wirkstoff in Form eines Granulats, z. B. mit Füllstoffen, wie Lactose, Bindemitteln, wie Stärken, und/oder Gleitmitteln, wie Talk oder Magnesiumstearat, und gegebenenfalls mit Stabilisatoren, enthalten. In weichen Kapseln ist der Wirkstoff vorzugsweise in geeigneten öligen Hilfsstoffen, wie fetten Ölen, Paraffinöl oder flüssigen Polyethylenglykolen, gelöst oder suspendiert, wobei ebenfalls Stabilisatoren und/oder antibakterielle Mittel zugefügt sein können. Den Tabletten oder Dragée-Überzügen und den Kapselhüllen können Farbstoffe oder Pigmente, z. B. zur Identifizierung oder zur Kennzeichnung verschiedener Wirkstoffdosen, Geschmackstoffe oder Süssmittel beigefügt werden.

Als pharmazeutische Präparate besonders bevorzugt sind durch Phospholipide stabilisierte Dispersionen des Wirkstoffes, vorzugsweise zur oralen Applikation, enthaltend

a) ein Phospholipid oder mehrere Phospholipide der Formel

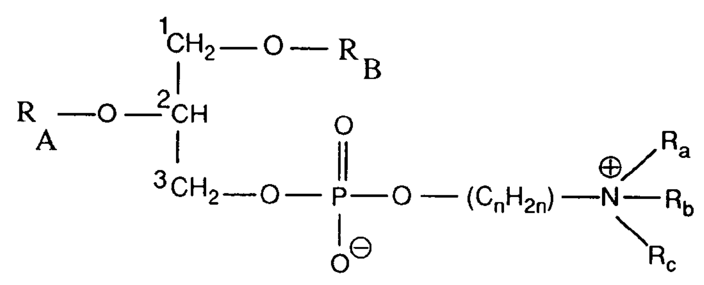

(A),

worin $R_A$ $C_{10-20}$-Acyl, $R_B$ Wasserstoff oder $C_{10-20}$-Acyl, $R_a$, $R_b$ und $R_c$ Wasserstoff oder $C_{1-4}$-Alkyl und n eine ganze Zahl von zwei bis vier darstellen, gewünschtenfalls b) ein weiteres Phospholipid oder mehrere weitere Phospholipide

c) den Wirkstoff und

d) eine pharmazeutisch annehmbare Trägerflüssigkeit und gewünschtenfalls weitere Hilfsstoffe und/oder Konservierungsmittel.

Das Herstellungsverfahren für diese Dispersionen ist dadurch gekennzeichnet, dass man eine Lösung oder Suspension der Komponenten a) und c) oder a), b) und c), vorzugsweise von a) und b) in einem Gewichtsverhältnis von 20 : 1 bis 1 : 5, insbesondere von 5 : 1 bis 1 : 1, durch Verdünnung mit Wasser in eine Dispersion umwandelt, anschliessend das organische Lösungsmittel entfernt, beispielsweise durch Zentrifugation, Gelfiltration, Ultrafiltration oder insbesondere durch Dialyse, z. B. tangentiale Dialyse, vorzugsweise gegen

Wasser, die erhaltene Dispersion, vorzugsweise nach Zugabe von Hilfsstoffen oder Konservierungsmitteln, erforderlichenfalls unter Einstellung eines annehmbaren pH-Wertes durch Zugabe von pharmazeutisch annehmbaren Puffern, wie Phosphatsalzen oder organischen Säuren (rein oder gelöst in Wasser), wie Essigssäure oder Zitronensäure, vorzugsweise zwischen pH 3 und 6, z. B. pH 4 - 5, falls sie nicht bereits die richtige Wirkstoffkonzentration hat, konzentriert, vorzugsweise auf eine Wirkstoffkonzentration von 2 bis 30 mg/ml, insbesondere von 10 bis 20 mg/ml, wobei die Konzentrierung vorzugsweise nach den zuletzt genannten Methoden zur Entfernung eines organischen Lösungsmittels erfolgt, insbesondere durch Ultrafiltration, z. B. unter Verwendung einer Apparatur zur Durchführung tangentialer Dialyse und Ultrafiltration.

Die nach diesem Verfahren herstellbare, durch Phospholipide stabilisierte Dispersion ist bei Zimmertemperatur mindestens mehrere Stunden stabil, reproduzierbar bezüglich des Mengenanteils der Komponenten und toxikologisch unbedenklich und daher insbesondere für die orale Applikation am Menschen geeignet.

Die Grössenordnung der erhaltenen Partikel in der Dispersion ist variabel und liegt vorzugsweise zwischen ca. $1{,}0 \times 10^{-8}$ bis ca. $1{,}0 \times 10^{-5}$ m, insbesondere zwischen etwa $10^{-7}$ und etwa $2 \times 10^{-6}$ m.

Die Nomenklatur der Phospholipide der Formel A und die Bezifferung der C-Atome erfolgt anhand der in Eur. J. of Biochem. 79, 11-21(1977) "Nomenclature of Lipids" von der IUPAC-IUB Commission on Biochemical Nomenclature (CBN) gegebenen Empfehlungen (sn-Nomenklatur, stereospecific numbering).

In einem Phospholipid der Formel A sind $R_A$ und $R_B$ mit den Bedeutungen $C_{10-20}$-Acyl vorzugsweise geradkettiges $C_{10-20}$-Alkanoyl mit einer geraden Anzahl an C-Atomen und geradkettiges $C_{10-20}$-Alkenoyl mit einer Doppelbindung und einer geraden Anzahl an C-Atomen.

Geradkettiges $C_{10-20}$-Alkanoyl $R_A$ und $R_B$ mit einer geraden Anzahl an C-Atomen sind beispielsweise n-Dodecanoyl, n-Tetradecanoyl, n-Hexadecanoyl oder n-Octadecanoyl.

Geradkettiges $C_{10-20}$-Alkenoyl $R_A$ und $R_B$ mit einer Doppelbindung und einer geraden Anzahl an C-Atomen sind beispielsweise 6-cis-, 6-trans-, 9-cis- oder 9-trans-dodecenoyl, -tetradecenoyl, -hexadecenoyl, -octadecenoyl oder -icosenoyl, insbesondere 9-cis-octadecenoyl (Oleoyl).

In einem Phospholipid der Formel A ist n eine ganze Zahl von zwei bis vier, vorzugsweise zwei. Die Gruppe der Formel $-(C_nH_{2n})-$ stellt unverzweigtes oder verzweigtes Alkylen dar, z.B. 1,1-Ethylen, 1,1-, 1,2- oder 1,3-Propylen oder 1,2-, 1,3- oder 1,4-Butylen. Bevorzugt ist 1,2-Eethylen (n=2).

Phospholipide der Formel A sind beispielsweise natürlich vorkommende Kephaline, worin $R_a$, $R_b$ und $R_c$ Wasserstoff bedeuten oder natürlich vorkommende Lecithine, worin $R_a$, $R_b$ und $R_c$ Methyl bedeuten, z.B. Kephalin oder Lecithin aus Sojabohnen, Rinderhirn, Rinderleber oder Hühnerei mit verschiedenen oder identischen Acylgruppen $R_A$ und $R_B$ oder Mischungen davon.

Bevorzugt sind synthetische, im wesentlichen reine Phospholipide der Formel A mit verschiedenen oder identischen Acylgruppen $R_A$ und $R_B$.

Der Begriff "synthetisches" Phospholipid der Formel A definiert Phospholipide, welche bezüglich $R_A$ und $R_B$ eine einheitliche Zusammensetzung haben. Solche synthetischen Phospholipide sind vorzugsweise die unten definierten Lecithine und Kephaline, deren Acylgruppen $R_A$ und $R_B$ eine definierte Struktur haben und von einer definierten Fettsäure mit einem Reinheitsgrad höher als ca. 95 % abgeleitet sind. $R_A$ und $R_B$ können gleich oder verschieden und ungesättigt oder gesättigt sein. Bevorzugt ist $R_A$ gesättigt, z.B. n-Hexadecanoyl, und $R_B$ ungesättigt, z.B. 9-cis-Octadecenoyl (= Oleoyl).

Der Begriff "natürlich vorkommende" Phospholipide der Formel A definiert Phospholipide, welche bezüglich $R_A$ und $R_B$ keine einheitliche Zusammensetzung haben. Solche natürlichen Phospholipide sind ebenfalls Lecithine und Kephaline, deren Acylgruppen $R_A$ und $R_B$ strukturell undefinierbar und von natürlich vorkommenden Fettsäuregemischen abgeleitet sind.

Der Begriff "im wesentlichen reines" Phospholipid definiert einen Reinheitsgrad von mehr als 70 % (Gew.) des Phospholipids der Formel A, welcher anhand geeigneter Bestimmungsmethoden, z.B. papierchromatographisch, nachweisbar ist.

Besonders bevorzugt sind synthetische, im wesentlichen reine Phospholipide der Formel A, worin $R_A$ die Bedeutung geradkettiges $C_{10-20}$-Alkanoyl mit einer geraden Anzahl an C-Atomen und $R_B$ die Bedeutung geradkettiges $C_{10-20}$-Alkenoyl mit einer Doppelbindung und einer geraden Anzahl an C-Atomen haben. $R_a$, $R_b$ und $R_c$ sind Methyl und n ist zwei.

In einem besonders bevorzugten Phospholipid der Formel A bedeuten $R_A$ n-Dodecanoyl, n-Tetradecanoyl, n-Hexadecanoyl oder n-Octadecanoyl und $R_B$ 9-cis-Dodecenoyl, 9-cis-Tetradecenoyl, 9-cis-Hexadecenoyl, 9-cis-Octadecenoyl oder 9-cis-Icosenoyl. $R_a$, $R_b$ und $R_c$ sind Methyl und n ist zwei.

Ein ganz besonders bevorzugtes Phospholipid der Formel A ist synthetisches 1-n-Hexadecanoyl-2-(9-cis-octadecenoyl)-3-sn-phosphatidylcholin mit einer Reinheit von mehr als 95 %.

Bevorzugte natürliche, im wesentlichen reine Phospholipide der Formel A sind insbesondere Lecithin (L-α-Phosphatidylcholin) aus Sojabohnen oder Hühnerei.

Für die Acylreste in den Phospholipiden der Formeln A sind auch die in Klammern angegebenen Bezeichnungen gebräuchlich:
9-cis-Dodecenoyl (Lauroleoyl), 9-cis-Tetradecenoyl (Myristoleoyl), 9-cis-Hexadecenoyl (Palmitoleoyl), 6-cis-Octadecenoyl (Petroseloyl), 6-trans-Octadecenoyl (Petroselaidoyl), 9-cis-Octadecenoyl(Oleoyl), 9-trans-Octadecenoyl (Elaidoyl), 11-cis-Octadecenoyl (Vaccenoyl), 9-cis-Icosenoyl (Gadoleoyl), n-Dodecanoyl (Lauroyl), n-Tetradecanoyl (Myristoyl), n-Hexadecanoyl (Palmitoyl), n-Octadecanoyl (Stearoyl), n-Icosanoyl (Arachidoyl).

Weitere Phospholipide sind vorzugsweise Ester von Phosphatidsäure (3-sn-Phosphatidsäure) mit den genannten Aclyresten, wie Phosphatidylserin und Phosphatidylethanolamin.

Schwerlösliche Wirkstoffe können auch als wasserlösliche, pharmazeutisch annehmbare Salze vorliegen, wie oben definiert.

In der Trägerflüssigkeit d) sind die Komponenten a), b) und c) oder a) und c) als Liposomen so enthalten, dass sich mehrere Tage bis Wochen keine Feststoffe oder feste Aggregate wie Mizellen zurückbilden und die Flüssigkeit mit den genannten Komponenten, gegebenenfalls nach Filtration, vorzugsweise oral, applizierbar ist.

In der Trägerflüssigkeit d) können pharmazeutisch annehmbare, nicht toxische Hilfsstoffe enthalten sein, z.B. wasserlösliche Hilfsstoffe welche zur Herstellung von isotonischen Bedingungen geeignet sind, z.B. ionische Zusätze wie Kochsalz oder nichtionische Zusätze (Gerüstbildner) wie Sorbit, Mannit oder Glucose oder wasserlösliche Stabilisatoren für die Liposomendispersion wie Lactose, Fructose oder Sucrose.

Zusätzlich zu den wasserlöslichen Hilfsstoffen können in der Trägerflüssigkeit für flüssige pharmazeutische Formulierungen verwendbare Emulgatoren, Netzmittel oder Tenside vorhanden sein, insbesondere Emulgatoren wie Ölsäure, nichtionische Tenside vom Typ Fettsäure-Polyhydroxyalkoholester wie Sorbitanmonolaurat, -oleat, -stearat oder -palmitat, Sorbitantristearat oder -trioleat, Polyoxyethylen-Addukte von Fettsäure-Polyhydroxyalkoholestern wie Polyoxyethylen-sorbitanmonolaurat, -oleat, -stearat, -palmitat, -tristearat oder -trioleat, Polyethylenglycol-Fettsäureester wie Polyoxyethylstearat, Polyethylenglycol-400-stearat, Polyethylenglycol-2000-stearat, insbesondere Ethylenoxid-Propylenoxid Blockpolymere vom Typ Pluronic® (Wyandotte Chem. Corp.) oder Synperonic® (ICI).

Bevorzugte Konservierungsmittel sind z. B. Antioxidantien, wie Ascorbinsäure, oder Microbizide, wie Sorbinsäure oder Benzoesäure.

Salben sind Öl-in-Wasser-Emulsionen, die bis zu 70 %, vorzugsweise jedoch 20 - 50 % Wasser oder wässrige Phase aufweisen. Als Fettphase kommen in erster Linie Kohlenwasserstoffe, z.B. Vaseline®, Paraffinöl oder Hartparaffine in Frage, die zur Verbesserung des Wasserbindungsvermögens vorzugsweise geeignete Hydroxyverbindungen wie Fettalkohole oder Ester davon, z.B. Cetylalkohol oder Wollwachsalkohole, wie Wollwachs, enthalten. Emulgatoren sind entsprechende lipophile Substanzen, wie Sorbitan-fettsäureester (Spans®), z.B. Sorbitanoleat und/oder Sorbitanisostearat. Zusätze zur Wasserphase sind z.B. Feuchthaltungsmittel, wie Polyalkohole, z.B. Glycerin, Propylenglykol, Sorbit und/oder Polyethylenglycol, oder wie Konservierungsmittel und Riechstoffe.

Fettsalben sind wasserfrei und enthalten als Grundlage insbesondere Kohlenwasserstoffe, z.B. Paraffin, Vaseline® oder Paraffinöl, ferner natürliche oder partialsynthetische Fette, z.B. Kokosfettsäuretriglycerid, oder vorzugsweise gehärtete Öle, z.B. hydriertes Erdnuss- oder Ricinusöl, ferner Fettsäurepartialester des Glycerins, z.B. Glycerinmono- und/oder -distearat, sowie z.B. die im Zusammenhang mit den Salben erwähnten, die Wasseraufnahme steigernden Fettalkohole, Emulgatoren und/oder Zusätze.

Crèmes sind Öl-in-Wasser-Emulsionen, die mehr als 50 % Wasser aufweisen. Als ölige Grundlage verwendet man in erster Linie Fettalkohole, z.B. Lauryl-, Cetyl- oder Stearylalkohol, Fettsäuren, z.B. Palmitin- oder Stearinsäure, flüssige bis feste Wachse, z.B. Isopropylmyristat, Wollwachs oder Bienenwachs, und/oder Kohlenwasserstoffe,z.B. Vaseline® (Petrolatum) oder Paraffinöl. Als Emulgatoren kommen oberflächenaktive Substanzen mit vorwiegend hydrophilen Eigenschaften in Frage, wie entsprechende nichtionische Emulgatoren, z.B. Fettsäureester von Polyalkoholen oder Ethylenoxyaddukte davon, wie Polyglycerinsäurefettsäureester oder Polyethylensorbitan-fettsäureester (Tweens®), ferner Polyoxyethylen-fettalkoholether oder -fettsäureester, oder entsprechende ionische Emulgatoren, wie Alkalimetallsalze von Fettalkoholsulfaten, z.B. Natriumlaurylsulfat, Natriumcetylsulfat oder Natriumstearylsulfat, die man üblicherweise in Gegenwart von Fettalkoholen, z.B. Cetylalkohol oder Stearylalkohol, verwendet. Zusätze zur Wasserphase sind u.a. Mittel, welche die Austrocknung der Crèmes vermindern, z.B. Polyalkohole, wie Glycerin, Sorbit, Propylenglykol und/oder Polyethylenglykole, ferner Konservierungsmittel und Riechstoffe.

Pasten sind Crèmes und Salben mit sekretabsorbierenden Puderbestandteilen, wie Metalloxiden, z.B. Titanoxid oder Zinkoxid, ferner Talk und/oder Aluminiumsilikate, welche die Aufgabe haben, vorhandene Feuchtigkeit oder Sekrete zu binden.

Schäume werden aus Druckbehältern verabreicht und sind in Aerosolform vorliegende flüssige Öl-in-Wasser-Emulsionen, wobei halogenierte Kohlenwasserstoffe, wie Chlorfluorniederalkane, z.B. Dichlordifluormethan und Dichlortetrafluorethan, oder vorzugsweise nichthalogenierte gasförmige Kohlenwasserstoffe, Luft, $N_2O$ oder Kohlendioxid als Treibgase verwendet werden. Als Ölphase verwendet man u.a. die oben bei Salben und Crèmes verwendeten, ebenso die dort genannten Zusätze.

Tinkturen und Lösungen weisen meist eine wässrig-ethanolische Grundlage auf, der u.a. Polyalkohole, z.B. Glycerin, Glykole, und/oder Polyethylenglykol, als Feuchthaltemittel zur Herabsetzung der Verdunstung, und rückfettende Substanzen, wie Fettsäureester mit niederen Polyethylenglykolen, d.h. im wässrigen Gemisch lösliche, lipophile Substanzen als Ersatz für die der Haut mit dem Ethanol entzogenen Fettsubstanzen, und, falls nötig, andere Hilfs- und Zusatzmittel beigegeben sind.

Besonders geeignet sind die in den Beispielen beschriebenen pharmazeutischen Präparate.

Die oben zur Definition von Verbindungen der Formel I verwendeten Begriffe haben im Rahmen des vorliegenden Textes vorzugsweise die folgenden Bedeutungen:

In der Beschreibung der vorliegenden Erfindung bedeutet der bei der Definition von Gruppen oder Resten, z. B. Niederalkyl, Niederalkoxycarbonyl etc., verwendete Ausdruck "Nieder", dass die so definierten Gruppen oder Reste, falls nicht ausdrücklich anders definiert, bis einschliesslich 7 und bevorzugt bis einschliesslich 4 C-Atome enthalten.

Gegebenenfalls vorhandene asymmetrische Kohlenstoffatome in den Substituenten $R_1$, $B_1$, $R_2$, $R_3$, $A_1$und/oder $A_2$, sowie in aus $R_4$ und $R_5$ gemeinsam mit dem bindenden Stickstoffatom gebildetem substituiertem Thiomorpholino oder Morpholino, können in der (R)-, (S)- oder (R,S)-Konfiguration vorliegen. Somit können die vorliegenden Verbindungen als Isomerengemische oder als reine Isomeren, insbesondere als Diastereomerengemische, Enantiomerenpaare oder reine Enantiomere vorliegen.

Die in der Beschreibung der vorliegenden Erfindung verwendeten allgemeinen Ausdrücke und Bezeichnungen haben vorzugsweise die folgenden Bedeutungen, wobei auf den verschiedenen Definitionsebenen von den vor- oder nachstehend aufgeführten Resten beliebige Kombinationen oder Einzelreste anstelle der allgemeinen Definitionen verwendet werden können:

Niederalkoxycarbonyl $R_1$ enthält vorzugsweise einen verzweigten Niederalkylrest, insbesondere einen sec- oder tert-Niederalkylrest, und ist z. B. Butoxycarbonyl, wie tert-Butoxycarbonyl oder Isobutoxycarbonyl. Besonders bevorzugt ist tert-Butoxycarbonyl.

Heterocyclylcarbonyl $R_1$ enthält insbesondere einen 5- oder 6-gliedrigen Heterocyclus, der 1 bis 3 Heteroatome, die unabhängig voneinander aus S, O oder N ausgewählt sind, enthält, ungesättigt oder ganz oder teilweise gesättigt ist und ein- oder bis zu dreifach benzanelliert, cyclopenta-, cyclohexa- oder cycloheptaanelliert ist, wobei die genannten anellierten Ringe ein weiteres Stickstoffatom als Heteroatom enthalten können, beispielsweise einen Heterocyclylrest ausgewählt aus Pyrrolyl, Furanyl, Thienyl, Imidazolyl, Pyrazolyl, Oxazolyl, Thiazolyl, Pyridyl, Pyrazinyl, Pyrimidinyl, Indolyl, Chinolyl, Isochinolyl, Chinoxalinyl, β-Carbolinyl und einem benzanellierten, cyclopenta-, cyclohexa- oder cyclohepta-anellierten Derivat dieser Reste, die auch ganz oder teilweise gesättigt vorliegen können, vorzugsweise teilweise gesättigt, oder ist ausgewählt aus Pyridylcarbonyl, z. B. Pyridyl-3-carbonyl, Morpholinylcarbonyl, z. B. Morpholinocarbonyl, und Benzofuranoyl, z. B. 3-Benzofuranoyl, sowie alternativ oder ergänzend hierzu Tetrahydroisochinolylcarbonyl, z. B. Tetrahydroisochinolyl-3-carbonyl, vorzugsweise Tetrahydroisochinolyl-3(S)-carbonyl.

Benzyloxycarbonyl $R_1$ ist unsubstituiert oder durch bis zu drei unabhängig voneinander aus Fluor, Haloniederalkyl, z. B. Trifluormethyl oder Pentafluorethyl, Niederalkanoyl, wie Acetyl, Propanoyl, Butyryl oder Pivaloyl, Sulfo, Niederalkylsulfonyl, z. B. Methylsulfonyl, Ethylsulfonyl, n-Propylsulfonyl oder iso-Propylsulfonyl, und Cyano ausgewählte Reste substituiert. Bevorzugt ist unsubstituiertes oder durch einen aus Fluor, Trifluormethyl, Sulfo, Methylsulfonyl, Ethylsulfonyl und Cyano ausgewählten Rest im Phenylring o-, m- oder p-substituiertes, insbesondere p-substituiertes Benzyloxycarbonyl, z. B. Benzyloxycarbonyl, Fluorphenylmethoxycarbonyl, wie p-Fluorphenylmethoxycarbonyl, Trifluormethylphenylmethoxycarbonyl, wie p-Trifluormethylphenylmethoxycarbonyl, Methylsulfonylphenylmethoxycarbonyl, wie p-Methylsulfonylphenylmethoxycarbonyl, oder Cyanophenylmethoxycarbonyl, wie p-Cyanophenylmethoxycarbonyl.

Heterocyclyloxycarbonyl $R_1$ enthält als Heterocyclyl insbesondere einen 5- oder 6-gliedrigen Heterocyclus, der 1 bis 3 Heteroatome, die unabhängig voneinander aus S, O oder N ausgewählt sind, enthält, ungesättigt oder ganz oder teilweise gesättigt ist und ein- oder bis zu dreifach benzanelliert, cyclopenta-, cyclohexa- oder cyclohepta-anelliert ist, wobei die genannten anellierten Ringe ein weiteres Stickstoffatom als Heteroatom enthalten können, beispielsweise einen Rest ausgewählt aus Pyrrolyl, Thienyl, Imidazolyl, Pyrazolyl, Oxazolyl, Thiazolyl, Pyrazinyl, Pyrimidinyl, Indolyl, Chinolyl, Isochinolyl, Chinoxalinyl, β-Carbolinyl und einem benzanellierten, cyclopenta-, cyclohexa- oder cyclohepta-anellierten Derivat dieser Reste, die auch ganz oder teilweise gesättigt vorliegen können, wobei die Heterocyclylreste über ein Ringkohlenstoffatom an den Sauerstoff des zugehörigen Oxycarbonylrestes gebunden sind, vorzugsweise ausgewählt aus Pyrrolyl, Thienyl, Imidazolyl, Pyrazolyl, Oxazolyl, Thiazolyl, Pyrazinyl, Pyrimidinyl, Indolyl, Chinolyl, Isochinolyl, Chinoxalinyl, β-Carbolinyl und einem ganz oder teilweise gesättigten Derivat dieser Reste, z. B. einem teilweise gesättigten Derivat dieser Reste oder Indol-3-yl-oxycarbonyl, Benzthiazol-6-yl-oxycarbonyl oder Chinol-8-yl-oxycarbonyl. In einer ganz besonders bevorzugten Variante der Definition von $R_1$ sind die unter die Definition der Substituenten Heterocyclyloxycarbonyl fallenden Reste auf allen Definitionsebenen nicht umfasst.

In den genannten Resten kann die bindende Carbonyl- auch durch eine Thiocarbonylgruppe ersetzt sein. Bevorzugt ist eine Carbonylgruppe.

Niederalkylsulfonyl $R_1$ ist vorzugsweise Methylsulfonyl, Ethylsulfonyl, n-Propylsulfonyl oder iso-Propylsulfonyl. Die Verbindungen der Formel I, worin $R_1$ Niederalkylsulfonyl bedeutet und die übrigen Reste die genannten Bedeutungen haben, können bei der Definition der Verbindungen der Formel I wegfallen, oder sie sind besonders bevorzugt.

Heterocyclylsulfonyl enthält als Heterocyclyl vorzugsweise einen der unter Heterocyclylcarbonyl $R_1$ genannten Heterocyclen, der unsubstituiert oder durch Niederalkyl, wie Methyl oder Ethyl, substituiert ist, wobei Heterocyclen bevorzugt sind, die wenigstens ein Stickstoffatom enthalten, welches an den Schwefel der Sulfonylgruppe gebunden ist, und ist insbesondere Piperidinosulfonyl, unsubstituiertes oder am nicht an den Sulfonyl-Schwefel gebundenen Stickstoff durch Niederalkyl, wie Methyl, substituiertes Piperazin-1-yl-sulfonyl, Pyrrolidin-1-yl-sulfonyl, Imidazolidin-1-yl-sulfonyl, Pyrimidin-1-ylsulfonyl, Chinolin-1-ylsulfonyl, Morpholinosulfonyl oder Thiomorpholinosulfonyl, vor allem Thiomorpholinosulfonyl oder Morpholinosulfonyl. Die Verbindungen der Formel I, worin $R_1$ Heterocyclylsulfonyl bedeutet und die übrigen Reste die genannten Bedeutungen haben, können bei der Definition der Verbindungen der Formel I wegfallen, oder sie sind besonders bevorzugt.

N-(Heterocylylniederalkyl)-N-niederalkyl-aminocarbonyl $R_1$ enthält als Heterocyclyl vorzugsweise einen der unter Heterocyclylcarbonyl $R_1$ genannten Heterocyclen, insbesondere Pyridyl, wie 2-, 3- oder 4-Pyridyl, Pyrazinyl, Pyrimidinyl, Morpholinyl, wie Morpholino, Thiomorpholinyl, wie Thiomorpholino, oder Chinolyl, wie 2- oder 3-Chinolyl, und ist insbesondere N-(Heterocyclylmethyl)-N-methyl-aminocarbonyl, z. B. N-(Pyridylmethyl)-N-methyl-aminocarbonyl, wie N-(2-Pyridylmethyl)-N-methylaminocarbonyl. Die Verbindungen der Formel I, worin $R_1$ N-(Heterocylylniederalkyl)-N-niederalkyl-aminocarbonyl bedeutet und die übrigen Reste die genannten Bedeutungen haben, können bei der Definition der Verbindungen der Formel I wegfallen, oder sie sind besonders bevorzugt.

Ein bivalentes Radikal $B_1$ einer α-Aminosäure, welches N-terminal mit $R_1$ und C-terminal mit der Aminogruppe am $R_2$-$CH_2$- tragenden Kohlenstoffatom verbunden ist, ist vorzugsweise ausgewählt aus Glycin (H-Gly-OH), Alanin (H-Ala-OH), Valin (H-Val-OH), Norvalin (α-Aminovaleriansäure), Leucin, (H-Leu-OH), Isoleucin (H-Ile-OH), Norleucin (α-Aminohexansäure, H-Nle-OH), Serin (H-Ser-OH), Homoserin (α-Amino-γ-hydroxy- buttersäure), Threonin (H-Thr-OH), Methionin (H-Met-OH), Cystein (H-Cys-OH), Prolin (H-Pro-OH), trans-3- und trans-4-Hydroxyprolin, Phenylalanin (H-Phe-OH), p-Fluorphenylalanin (H-(p-F-Phe)-OH), Tyrosin (H-Tyr-OH), p-Methoxy-phenylalanin (H-(p-$CH_3O$-Phe)-OH), 4-Aminophenylalanin, 4-Chlorphenylalanin, 4-Carboxyphenylalanin, β-Phenylserin (β-Hydroxyphenylalanin), Phenylglycin, α-Naphthylalanin (H-Nal-OH), Cyclohexylalanin (H-Cha-OH), Cyclohexylglycin, Tryptophan (H-Trp-OH), Indolin-2-carbonsäure,1,2,3,4-Tetrahydroisochinolin-3-carbonsäure, Aminomalonsäure, Aminomalonsäure-monoamid, Asparaginsäure (H-Asp-OH), Asparagin (H-Asn-OH), Glutaminsäure (H-Glu-OH), Glutamin (H-Gln-OH), Histidin (H-His-OH), Arginin (H-Arg-OH), Lysin (H-Lys-OH), δ-Hydroxylysin, Ornithin (α,δ-Diaminovaleriansäure), α,γ-Diaminobuttersäure und α,β-Diaminopropionsäure, oder alternativ und ergänzend hierzu 4-Cyano-phenylalanin (H-(p-CN-Phe)-OH), besonders bevorzugt der Rest einer hydrophoben Aminosäure, z. B. Prolin, Phenylalanin, p-Fluorphenylalanin, p-Methoxyphenylalanin, Tyrosin, Phenylglycin, α-Naphthylalanin, Cyclohexylalanin, Cyclohexylglycin oder eine aliphatische α-Aminosäure ausgewählt aus Glycin, Valin, Norvalin, Alanin, Leucin, Norleucin und Isoleucin, insbesondere Valin, wobei jede der genannten α-Aminosäuren in der D-, L- oder (D,L)-, vorzugsweise in der L-Form vorliegt, und insbesondere mit Resten $R_1$ ausgewählt aus Niederalkoxycarbonyl, z. B. tert-Butoxycarbonyl, oder Heterocyclylcarbonyl, z. B. Morpholinocarbonyl, verknüpft ist.

Wenn $B_1$ eine Bindung bedeutet, ist $R_1$ direkt mit dem Aminostickstoff verbunden, den das den Rest $R_2$-$CH_2$- tragende Kohlenstoffatom in Formel I bindet.

Phenyl oder Cyclohexyl $R_2$ oder $R_3$ ist unsubstituiert oder durch bis zu drei unabhängig aus Hydroxy, Niederalkoxy, wie Methoxy oder Ethoxy, Halo, z. B. Fluor, Haloniederalkyl, z. B. Trifluormethyl, Sulfo, Niederalkylsulfonyl, z. B. Methyl- oder Ethylsulfonyl, Cyano und Nitro ausgewählte Reste substituiert, vorzugsweise durch ein bis zwei dieser Reste, besonders bevorzugt ausgewählt aus Hydroxy, Methoxy, Fluor, Trifluormethyl, Sulfo, Niederalkylsulfonyl, z. B. Methyl- oder Ethylsulfonyl, und Cyano; für Phenyl ganz besonders bevorzugt aus Fluor und Cyano, für Cyclohexyl ganz besonders bevorzugt aus Fluor, Trifluormethyl, Sulfo oder Niederalkylsulfonyl, in erster Linie Fluor; wobei die genannten Substituenten in 2-, 3- oder 4- Stellung des Phenyl- oder Cyclohexylringes, insbesondere in 4-Stellung, gebunden sind, wie in Phenyl, Cyclohexyl, 4-Fluor- oder 4-Cyanophenyl oder 4-Fluorcyclohexyl, insbesondere in Phenyl, Cyclohexyl, 4-Cyano-phenyl oder 4-Fluorphenyl.

Besonders bevorzugt sind solche Kombinationen von $R_2$ und $R_3$, bei denen wenigstens einer der Reste $R_2$ oder $R_3$ durch einen oder bis zu drei aus Halo, insbesondere Fluor, Haloniederalkyl, insbesondere Trifluormethyl, Sulfo, Niederalkylsulfonyl, insbesondere Methyl oder Ethylsulfonyl, Cyano und Nitro ausgewählte Reste substituiert ist, wobei in stark bevorzugter Weise ein Substituent ausgewählt aus Fluor oder Cyano vorliegt.

Noch stärker bevorzugt ist $R_2$ ausgewählt aus Phenyl, 4-Hydroxyphenyl, 4-Methoxyphenyl, 4-Fluorphenyl, Cyclohexyl und 4-Trifluormethylphenyl, während $R_3$ ausgewählt ist aus Phenyl, 4-Hydroxyphenyl, 4-Methoxyphenyl, Cyclohexyl, 4-Fluorphenyl, 4-Trifluormethylphenyl und 4-Cyanophenyl.

In erster Linie ist $R_2$ ausgewählt aus Phenyl, 4-Fluorphenyl und Cyclohexyl, während $R_3$ ausgewählt ist aus Phenyl, Cyclohexyl, 4-Fluorphenyl, 4-Methoxyphenyl und 4-Cyanophenyl.

In allererster Linie bevorzugt sind die Kombinationen: $R_2$ Phenyl und $R_3$ Phenyl; $R_2$ Cyclohexyl und $R_3$ 4-Cyanophenyl; $R_2$ Cyclohexyl und $R_3$ 4-Fluorphenyl; und $R_2$ und $R_3$ jeweils Cyclohexyl. Alternativ oder ergänzend hierzu sind auch die Kombinationen $R_2$ Phenyl und $R_3$ 4-Fluorphenyl; $R_2$ Phenyl und $R_3$ 4-Cyanophenyl; $R_2$ 4-Fluorphenyl und $R_3$ 4-Fluorphenyl; $R_2$ 4-Fluorphenyl und $R_3$ 4-Trifluormethylphenyl; $R_2$ 4-Trifluormethylphenyl und $R_3$ Phenyl; $R_2$ 4-Trifluormethylphenyl und $R_3$ 4-Fluorphenyl; $R_2$ 4-Trifluormethylphenyl und $R_3$ 4-Trifluormethylphenyl; $R_2$ Hydroxyphenyl und $R_3$ Phenyl; $R_2$ Phenyl und $R_3$ Hydroxyphenyl; $R_2$ Phenyl und $R_3$ 4-Methoxyphenyl; $R_2$ Hydroxyphenyl und $R_3$ Hydroxyphenyl; oder $R_2$ Cyclohexyl und $R_3$ 4-Methoxyphenyl in allererster Linie bevorzugt.

Hydroxygruppen, insbesondere die Hydroxygruppe in Verbindungen der Formel I an dem Kohlenstoffatom, welches dem Kohlenstoffatom benachbart ist, das den Rest $R_2$-$CH_2$-trägt, können frei oder in geschützter Form vorliegen, insbesondere frei oder geschützt als phyiologisch spaltbare Ester, z. B. als Niederalkanoyloxy, wie Acetyloxy. Vorzugsweise ist die zuletzt genannte Hydroxygruppe frei.

Ein bivalentes Radikal einer $\alpha$-Aminosäure $A_1$, welches N-terminal mit der Gruppe -C=O und C-terminal mit $A_2$ verbunden ist, ist beispielsweise eine der oben für $B_1$ genannten $\alpha$-Aminosäuren, wobei diese Aminosäuren in der (D)-, (L)- oder (D,L)-Form, vorzugsweise der (D)- oder (L)-, insbesondere der (L)-Form, vorliegen können. Bevorzugt sind die unter $B_1$ genannten hydrophoben $\alpha$-Aminosäuren, insbesondere die dort genannten aliphatischen hydrophoben $\alpha$-Aminosäuren, z. B. Glycin, Valin oder Isoleucin. In den genannten $\alpha$-Aminosäuren ist die an $A_2$ bindende Carboxygruppe nicht reduziert oder ferner reduziert, insbesondere zu einer Methylengruppe, z. B. in den genannten hydrophoben $\alpha$-Aminosäuren, wie in den reduzierten Aminosäureradikalen Gly(red), Val(red) oder Ile(red), insbesondere in Val(red), wobei der Zusatz (red) die Reduktion der Carbonylgruppe des entsprechenden Aminosäureradikals zur Methylengruppe anzeigt.

Bedeutet $A_1$ eine Bindung, so ist $A_2$ direkt mit der Carbonylgruppe am Kohlenstoffatom, der den Rest $R_3$-$CH_2$- trägt, verbunden.

Ein bivalentes Radikal einer $\alpha$-Aminosäure $A_2$, welches N-terminal mit $A_1$ und C-terminal mit der Gruppe $NR_4R_5$ verbunden ist, ist beispielsweise eine der oben für $B_1$ genannten $\alpha$-Aminosäuren, wobei diese Aminosäuren in der (D)-, (L)- oder (D,L)-Form, vorzugsweise der (D)- oder (L)-, insbesondere der (L)-Form, vorliegen können. Bevorzugt sind die unter $B_1$ genannten hydrophoben $\alpha$-Aminosäuren, z. B. Glycin, Valin, Phenylalanin, p-Fluorphenylalanin, Tyrosin, p-Methoxy-phenylalanin, Phenylglycin, $\alpha$-Naphthylalanin, Cyclohexylalanin oder Cyclohexylglycin, vorzugsweise Glycin, Valin, Phenylalanin, p-Fluorphenylalanin, p-Methoxy-phenylalanin oder Cyclohexylalanin, wobei die genannten Reste in der (D)- oder (L)-Form, vorzugsweise, ausser Phenylalanin, das in der (L)- oder der (D)-Form vorliegt, in der (L)-Form vorliegen.

Ein aus $A_1$ und $A_2$ gebildetes bivalentes Radikal eines Dipeptides, dessen zentrale Peptidbindung reduziert ist und das N-terminal mit der Gruppe -C=O und C-terminal mit der Gruppe $NR_4R_5$ verbunden ist, besteht vorzugsweise aus 2 der oben genannten hydrophoben $\alpha$-Aminosäuren, insbesondere aus einem N-terminalen Aminosäureradikal ausgewählt aus Gly(red), Val(red) und Ile(red) und einer C-terminalen Aminosäure ausgewählt aus Glycin, Phenylalanin, Tyrosin, p-Methoxyphenylalanin, Cyclohexylalanin und p-Fluorphenylalanin.

Besonders bevorzugt bilden $A_1$ und $A_2$ zusammen ein bivalentes Radikal eines Dipeptides der Formel Val-Phe, Ile-Phe, Val-Cha, Ile-Cha, Val-Gly, Val-(p-F-Phe), Val-(p-$CH_3O$-Phe), Gly-(p-F-Phe); und alternativ oder zusätzlich eines Dipeptides der Formel Val-Tyr, Ile-Tyr, Gly-Tyr, Ile-Gly oder Val-Val; worin die Aminosäuren in der (D)- oder (L)-, insbesondere der (L)-Form vorliegen mit Ausnahme von (L)-Val-Phe, in dem Phe in der (L)- oder (D)-Form vorliegt, oder eines Derivates hiervon mit reduzierter zentraler Amidbindung, z. B. mit der Formel Val(red)-Phe, das N-terminal mit der Gruppe -C=O und C-terminal mit der Gruppe $NR_4R_5$ verbunden ist.

Eine bevorzugte Ausführungsform der Erfindung bezieht sich entweder auf die Verbindungen der Formel I, in denen $B_1$ eines der genannten bivalenten Radikale einer $\alpha$-Aminosäure bedeutet und einer der Reste $A_1$ oder $A_2$ eine Bindung bedeutet und der andere eine der genannten $\alpha$-Aminosäuren bedeutet, oder auf diejenigen Verbindungen der Formel I, worin $B_1$ eine Bindung bedeutet und $A_1$ und $A_2$ jeweils eines der genannten bivalenten Radikale einer $\alpha$-Aminosäure oder gemeinsam eines der genannten bivalenten Radikale eines Dipeptides mit reduzierter zentraler Amidbindung bedeuten.

Aus $R_4$ und $R_5$ gemeinsam mit dem bindenden Stickstoffatom gebildetes Thiomorpholino oder Morpholino ist unsubstituiert oder substituiert an einem oder mehreren der Kohlenstoffatome, bevorzugt einem Kohlenstoffatom, durch Niederalkyl, wie Ethyl, Propyl, Butyl, iso-Butyl oder tert-Butyl, durch Phenyl- oder Naphthylniederalkyl, wie Benzyl, 1- oder 2-Naphthylmethyl oder Phenyl-1- oder Phenyl-2-ethyl, insbesondere Phenyl-1- oder Phenyl-2-ethyl, durch Hydroxy, durch Niederalkoxy, wie Methoxy, Ethoxy oder tert-Butoxy, durch Amino, durch Niederalkylamino, wie Methyl- oder Ethylamino, oder durch Diniederalkylamino, wie Dimethylamino oder Diethylamino, durch Niederalkanoyl, wie Acetyl oder Propionyl, durch Phenyl- oder Naphthyl-niederalkanoyl, wie Phenylacetyl oder 1-oder 2-Naphthylacetyl, durch Carboxy, durch Niederalkoxycarbonyl, wie iso-Propoxycarbonyl oder tert-Butoxycarbonyl, durch Phenyl-, Naphthyl- oder Fluorenylniederalkoxycarbonyl, wie Benzyloxycarbonyl, 1- oder 2-Naphthylmethoxycarbonyl oder 9-Fluorenylmethoxycarbonyl, durch Carbamoyl, durch Mono- oder Diniederalkylcarbamoyl, wie Dimethylcarbamoyl, durch Mono- oder Di-hydroxyniederalkyl-carbamoyl, wie Dihydroxymethyl-carbamoyl, durch Sulfo, durch Niederalkylsulfonyl, wie Methylsulfonyl oder Ethylsulfonyl, durch Phenyl- oder Naphthylsulfonyl, wobei Phenyl durch Niederalkyl, z. B. Methyl oder Ethyl, substituiert sein kann, z. B. Phenylsulfonyl oder Toluolsulfonyl, durch Sulfamoyl, durch Halogen, z. B. Fluor oder Chlor, durch Cyano, durch Nitro und/oder durch Oxo.

Sehr bevorzugt bilden $R_4$ und $R_5$ zusammen mit dem bindenden Stickstoffatom unsubstituiertes Thiomorpholino oder Morpholino, vor allem unsubstituiertes Morpholino.

Salze von Hemmstoffen von HIV-Aspartatproteasen, insbesondere von Verbindungen der Formel I, sind insbesondere Säureadditionssalze, Salze mit Basen oder bei Vorliegen mehrerer salzbildender Gruppen gegebenenfalls auch Mischsalze oder innere Salze.

Salze sind in erster Linie die pharmazeutisch verwendbaren, nichttoxischen Salze, insbesondere von Verbindungen der Formel I.

Solche Salze werden beispielsweise von Hemmstoffen von HIV-Aspartatproteasen, insbesondere Verbindungen der Formel I mit einer sauren Gruppe, z. B. einer Carboxy- oder Sulfogruppe, gebildet und sind beispielsweise deren Salze mit geeigneten Basen, wie nicht-toxische, von Metallen der Gruppe Ia, Ib, IIa und IIb des Periodensystems der Elemente abgeleitete Metallsalze, in erster Linie geeignete Alkalimetall-, z. B. Lithium-, Natrium- oder Kalium-, oder Erdalkalimetallsalze, z. B. Magnesium- oder Calciumsalze, ferner Zinksalze oder Ammoniumsalze, auch solche Salze, welche mit organischen Aminen, wie gegebenenfalls durch Hydroxy substituierten Mono-, Di- oder Trialkylaminen, insbesondere Mono-, Di- oder Triniederalkylaminen, oder mit quaternären Ammoniumverbindungen gebildet werden, z. B. mit N-Methyl-Nethylamin, Diethylamin, Triethylamin, Mono-, Bis- oder Tris-(2-hydroxyniederalkyl)-aminen, wie Mono-, Bis- oder Tris-(2-hydroxyethyl)-amin, 2-Hydroxy-tertbutylamin oder Tris(hydroxymethyl)methylamin, N,N-Diniederalkyl-N-(hydroxyniederalkyl)-aminen, wie N,N-Dimethyl-N-(2-hydroxyethyl)-amin oder Tri-(2-hydroxyethyl)amin, N-Methyl-D-glucamin oder quaternären Ammoniumsalzen, wie Tetrabutylammoniumsalzen. Die Verbindungen der Formel I mit einer basischen Gruppe, z. B. einer Aminogruppe, können Säureadditionssalze bilden, beispielsweise mit anorganischen Säuren, z. B. Halogenwasserstoffsäure, wie Salzsäure, Schwefelsäure oder Phosphorsäure, oder mit organischen Carbon-, Sulfon-, Sulfo- oder Phosphosäuren oder N-substituierter Sulfaminsäuren, wie z. B. Essigsäure, Propionsäure, Glykolsäure, Bernsteinsäure, Maleinsäure, Hydroxymaleinsäure, Methylmaleinsäure, Fumarsäure, Äpfelsäure, Weinsäure, Gluconsäure, Glucarsäure, Glucuronsäure, Zitronensäure, Benzoesäure, Zimtsäure, Mandelsäure, Salicylsäure, 4-Aminosalicylsäure, 2-Phenoxybenzoesäure, 2-Acetoxybenzoesäure, Embonsäure, Nicotinsäure oder Isonicotinsäure, ferner mit Aminosäuren, wie z. B. den weiter vorn genannten $\alpha$-Aminosäuren, sowie mit Methansulfonsäure, Ethansulfonsäure, 2-Hydroxyethansulfonsäure, Ethan-1,2-disulfonsäure, Benzolsulfonsäure, 4-Methylbenzolsulfonsäure, Naphthalin-2-sulfonsäure, 2- oder 3-Phosphoglycerat, Glucose-6-phosphat, N-Cyclohexylsulfaminsäure (unter Bildung der Cyclamate) oder mit anderen sauren organischen Verbindungen, wie Ascorbinsäure. Verbindungen der Formel I mit sauren und basischen Gruppen können auch innere Salze bilden.

Zur Isolierung oder Reinigung bei Zwischenschritten, beispielsweise bei der Herstellung von pharmazeutischen Präparaten, können auch pharmazeutisch ungeeignete Salze Verwendung finden.

Die Ausdrücke "Verbindungen" und "Salze" schliessen ausdrücklich auch einzelne Verbindungen oder einzelne Salze ein.

Soweit vor- und nachstehend von Hemmstoffen von HIV-Aspartatproteasen, insbesondere der Formel I, (auch als "Wirksubstanzen", "Verbindungen" etc.) die Rede ist, sind, soweit sinnvoll und zweckmässig, auch die entsprechenden pharmazeutisch annehmbaren Salze, sofern salzbildende Gruppen vorliegen, gemeint.

Vorzugsweise betrifft die Erfindung pharmazeutische Präparate enthaltend Verbindungen der Formel I oder die Verwendung oder ein Verfahren oder eine Methode zur Behandlung unter Verabreichung von Verbindungen der Formel I, worin $R_1$ Wasserstoff, tert-Butoxycarbonyl, Isobutyloxycarbonyl, Pyridin-3-carbonyl, Morpholinocarbonyl, 3-Benzofuranoyl, 1,2,3,4-Tetrahydro-isochinolin-3-carbonyl, mit bis zu drei unabhängig voneinander aus Fluor, Haloniederalkyl, Niederalkanoyl, Sulfo, Niederalkylsulfonyl und Cyano ausgewählten Resten substituiertes Benzyloxycarbonyl oder Heterocyclyloxycarbonyl bedeutet, worin Heterocyclyl über ein Kohlenstoffatom gebunden ist und ausgewählt ist aus Pyrrolyl, Thienyl, Imidazolyl, Pyrazolyl, Oxazolyl, Thiazolyl, Pyrazinyl, Pyrimidinyl, Indolyl, Chinolyl, Isochinolyl, Chinoxalinyl, β-Carbolinyl und einem ganz oder teilweise gesättigten Derivat dieser Reste, oder worin die Bedeutung Heterocyclyloxycarbonyl für $R_1$ fehlt, $B_1$ eine Bindung oder ein zweiwertiges Radikal einer α-Aminosäure bedeutet, welches N-terminal mit $R_1$ und C-terminal mit der Aminogruppe am $R_2$-$CH_2$- tragenden Kohlenstoffatom verbunden ist, bevorzugt den Rest einer hydrophoben Aminosäure, z. B. Prolin, Phenylalanin, p-Fluorphenylalanin, Phenylglycin, α-Naphthylalanin, Cyclohexylalanin, Cyclohexylglycin oder einer aliphatischen α-Aminosäure ausgewählt aus Glycin, Valin, Norvalin, Alanin, Leucin, Norleucin und Isoleucin, insbesondere Valin, wobei vorzugsweise jede der genannten α-Aminosäuren in der D-, L- oder (D,L)-, vorzugsweise in der L-Form vorliegt, bedeutet, wobei vorzugsweise jede der genannten Aminosäuren mit einem der unter $R_1$ genannten Reste ausgewählt aus Wasserstoff, N-tert-Butoxycarbonyl oder Morpholinocarbonyl substituiert ist, $R_2$ und $R_3$ unabhängig voneinander Phenyl oder Cyclohexyl, wobei diese Reste unsubstituiert oder durch ein bis zwei unabhängig voneinander aus Hydroxy, Methoxy, Fluor, Sulfo, Niederalkylsulfonyl, Trifluormethyl und Cyano ausgewählte Reste substituiert sind, wie oben in den allgemeinen Definitionen gezeigt, bedeuten, $A_1$ ein bivalentes Radikal einer hydrophoben α-Aminosäure, wie oben unter den allgemeinen Definitionen gezeigt, bedeutet, welches N-terminal mit der Gruppe -C=O und C-terminal mit $A_2$ verbunden ist, $A_2$ ein bivalentes Radikal einer hydrophoben α-Aminosäure, vorzugsweise wie oben unter den allgemeinen Definitionen definiert, bedeutet, welches N-terminal mit A1 verbunden ist und C-terminal mit dem Rest $NR_4R_5$ verbunden ist, wobei die genannten Aminosäurereste in der (D)- oder (L)-Form, vorzugsweise, ausser Phenylalanin, das in der (L)- oder der (D)-Form vorliegt, in der (L)-Form vorliegen, insbesondere $A_1$ und $A_2$ ein bivalentes Radikal eines Dipeptides der Formel Val-Phe, Ile-Phe, Val-Cha, Ile-Cha, Ile-Gly, Val-Val, Val-Gly, Val-(p-F-Phe), Val-Tyr, Val-(p-$CH_3O$-Phe) oder Gly-(p-F-Phe) bilden, worin die Aminosäuren in der (D)- oder (L)-, insbesondere der (L)-Form vorliegen mit Ausnahme von (L)-Val-Phe, in dem Phe in der (L)- oder (D)-Form vorliegt; oder $A_1$ und $A_2$ zusammen ein bivalentes Radikal eines Dipeptides aus zwei, vorzugsweise der oben unter den allgemeinen Definitionen genannten, hydrophoben α-Aminosäuren bilden, dessen zentrale Amidbindung reduziert ist und das N-terminal mit der Gruppe -C=O und C-terminal mit der Gruppe $NR_4R_5$ verbunden ist, wie in den allgemeinen Definitionen aufgeführt, z. B. mit der Formel Val(red)-Phe, und $R_4$ und $R_5$ gemeinsam mit dem bindenden Stickstoffatom Thiomorpholino oder Morpholino, insbesondere Morpholino, bedeuten; und alternativ oder ergänzend hierzu die Verbindungen der Formel I, worin $R_1$ Morpholinosulfonyl oder N-(2-Pyridylmethyl)-N-methyl-aminocarbonyl bedeutet und die übrigen Reste die genannten Bedeutungen haben; und von pharmazeutisch verwendbaren Salzen dieser Verbindungen, sofern salzbildende Gruppen vorliegen; wobei die Hydroxygruppe in Verbindungen der Formel I an dem Kohlenstoffatom, welches dem Kohlenstoffatom benachbart ist, das den Rest $R_2$-$CH_2$- trägt, frei oder in durch Niederalkanoyl geschützter Form vorliegt, insbesondere in freier Form; und wobei bei der Definition von $R_1$ Heterocyclyloxycarbonyl auch wegfallen kann.

In erster Linie betrifft die vorliegende Erfindung pharmazeutische Präparate enthaltend Verbindungen der Formel I oder die Verwendung oder ein Verfahren oder eine Methode zur Behandlung unter Verabreichung von Verbindungen der Formel I, worin $R_1$ Wasserstoff, tert-Butoxycarbonyl, Isobutyloxycarbonyl, Pyridin-3-carbonyl, Morpholinocarbonyl, 3-Benzofuranoyl oder 1,2,3,4-Tetrahydro-isochinolin-3-carbonyl bedeutet, oder alternativ oder ergänzend hierzu Morpholinosulfonyl oder N-(2-Pyridylmethyl)-N-methyl-aminocarbonyl bedeutet, $B_1$ eine Bindung oder ein bivalentes Radikal der α-Aminosäure Valin bedeutet, welches N-terminal mit $R_1$ und C-terminal mit der Aminogruppe am $R_2$-$CH_2$- tragenden Kohlenstoffatom verbunden ist, wobei $R_1$ im letzteren Falle bevorzugt Wasserstoff, tert-Butoxycarbonyl oder Morpholinocarbonyl bedeutet oder alternativ oder ergänzend hierzu Morpholinosulfonyl oder N-(2-Pyridylmethyl)-N-methyl-aminocarbonyl bedeutet, $R_2$ und $R_3$ unabhängig voneinander Phenyl oder Cyclohexyl bedeuten, wobei diese Reste unsubstituiert oder durch ein bis zwei unabhängig voneinander aus Hydroxy, Methoxy, Fluor und Cyano ausgewählte Reste substituiert sind, insbesondere durch einen der genannten Reste, vorzugsweise in 4-Stellung, z. B. in 4-Hydroxyphenyl, 4-Methoxyphenyl, 4-Fluorphenyl, 4-Cyanophenyl oder 4-Fluorcyclohexyl, wie in den Kombinationen von $R_2$ und $R_3$, die unter den allgemeinen Definitionen oben als in erster Linie bevorzugt genannt sind, oder alternativ oder ergänzend hierzu $R_2$ und $R_3$ unabhängig voneinander Phenyl oder Cyclohexyl, welches unsubstituiert oder durch ein bis zwei unabhängig voneinander aus Trifluormethyl, Cyano und Fluor ausgewählte Reste substituiert ist, insbesondere durch einen dieser Reste, vorzugsweise in 4-Stellung, z. B. in 4-Trifluormethylphenyl, 4-Cyanophenyl oder 4-Fluorphenyl, bedeuten, $A_1$ und $A_2$ zusammen ein bivalentes Radikal eines Dipeptides der Formel Val-Phe, Ile-Phe, Val-Cha, Ile-Cha, Ile-Gly, Val-Val, Val-Gly, Val-(p-F-Phe), Val-Tyr, Val-(p-$CH_3O$-Phe), Gly-(p-F-Phe) oder eines Derivates hiervon mit reduzierter zentraler Amidbindung der Formel Val(red)-Phe bilden, das N-terminal mit der Gruppe -C=O und C-terminal mit der Gruppe $NR_4R_5$ verbunden ist, und $R_4$ und $R_5$ gemeinsam mit dem bindenden Stickstoffatom Thiomorpholino oder Morpholino, insbesondere Morpholino, bedeuten, und von pharmazeutisch verwendbaren Salzen dieser Verbindungen, sofern salzbildende Gruppen vorliegen, wobei die die Hydroxygruppe in Verbindungen der Formel I an dem Kohlenstoffatom, welches dem Kohlenstoffatom benachbart ist, das den Rest $R_2$-$CH_2$- trägt, frei oder in durch Acetyl geschützter Form vorliegt, wobei sowohl die freien Verbindungen der Formel I als auch die geschützte Form, worin alle weiteren Reste die genannten Bedeutungen haben, oder deren Salze, besonders bevorzugt sind.

Sehr wichtig sind pharmazeutische Präparate enthaltend Verbindungen der Formel I oder die Verwendung oder ein Verfahren oder eine Methode zur Behandlung unter Verabreichung der Verbindung der Formel I, worin $R_1$ Niederalkoxycarbonyl, insbesondere sekundäres oder tertiäres Niederalkoxycarbonyl oder Methoxycarbonyl, $B_1$ eine Bindung, $R_2$ und $R_3$ Phenyl, $A_1$ Valyl, insbesondere (L)-Valyl, $A_2$ Phenylalanyl, insbesondere (L)-Phenylalanyl, und $R_4$ und $R_5$ zusammen mit dem bindenden Stickstoffatom Morpholino bedeuten.

Ganz besonders wichtig sind pharmazeutische Präparate enthaltend Verbindungen der Formel I oder die Verwendung oder ein Verfahren oder eine Methode zur Behandlung unter Verabreichung der Verbindung der Formel I, worin $R_1$ tert-Butoxycarbonyl, $B_1$ eine Bindung, $R_2$ und $R_3$ Phenyl, $A_1$ Valyl, insbesondere (L)-Valyl, $A_2$ Phenylalanyl, insbesondere (L)-Phenylalanyl, und $R_4$ und $R_5$ zusammen mit dem bindenden Stickstoffatom Morpholino bedeuten.

Ganz besonders wichtig sind pharmazeutische Präparate enthaltend Verbindungen der Formel I oder die Verwendung oder ein Verfahren oder eine Methode zur Behandlung unter Verabreichung der Verbindung der Formel I, worin $R_1$ tert-Butoxycarbonyl, $B_1$ eine Bindung, $R_2$ Phenyl, $R_3$ 4-Methoxyphenyl, $A_1$ Valyl, insbesondere (L)-Valyl, $A_2$ Phenylalanyl, insbesondere (L)-Phenylalanyl, und $R_4$ und $R_5$ zusammen mit dem bindenden Stickstoffatom Morpholino bedeuten.

Ganz besonders wichtig sind pharmazeutische Präparate enthaltend Verbindungen der Formel I oder die Verwendung oder ein Verfahren oder eine Methode zur Behandlung unter Verabreichung der Verbindung der Formel I, worin $R_1$ tert-Butoxycarbonyl, $B_1$ eine Bindung, $R_2$ Cyclohexyl, $R_3$ 4-Methoxyphenyl, $A_1$ Valyl, insbesondere (L)-Valyl, $A_2$ Phenylalanyl, insbesondere (L)-Phenylalanyl, und $R_4$ und $R_5$ zusammen mit dem bindenden Stickstoffatom Morpholino bedeuten.

Am allerwichtigsten sind pharmazeutische Präparate enthaltend Verbindungen der Formel I oder die Verwendung oder ein Verfahren oder eine Methode zur Behandlung unter Verabreichung der in den Beispielen genannten Verbindungen der Formel I, vorzugsweise unter den dort genannten Bedingungen.

Die nachfolgenden Beispiele dienen der Illustration der Erfindung, ohne ihren Umfang in irgendeiner Weise einzuschränken:

Temperaturen werden in Celsiusgraden (°C) angegeben. Sofern keine Temperatur angegeben ist, erfolgt die Reaktion bei Raumtemperatur. Die $R_f$- Werte, die das Verhältnis von Laufstrecke der jeweiligen Substanz zur Laufstrecke der Laufmittelfront angeben, werden auf Kieselgeldünnschichtplatten durch Dünnschichtchromatographie (DC) in folgenden Lösungsmittelsystemen ermittelt:

DC-Laufmittelsysteme:

| | | |
|---|---|---|
| A | Hexan/Essigsäureethylester | 1:1 |
| B | Essigsäureethylester | - |
| C | Hexan/Essigsäureethylester | 4:1 |
| D | Hexan/Essigsäureethylester | 2:1 |
| E | Hexan/Essigsäureethylester | 3:1 |
| F | Methylenchlorid/Methanol | 9:1 |
| G | Chloroform/Methanol/Wasser/Eisessig | 85:13:1,5:0,5 |
| H | Hexan/Essigsäureethylester | 1:2 |

Die Abkürzung "$R_f$(B)" bedeutet beispielsweise, dass der $R_f$- Wert im Lösungsmittelsystem B ermittelt wurde. Das Mengenverhältnis von Lösungsmitteln zueinander ist stets in Volumenanteilen (v/v) angegeben. Auch bei der Definition der Fliessmittel-Systeme für die Säulenchromatographie werden die Mengenverhältnisse der verwendeten Lösungsmittel in Volumenanteilen (v/v) angegeben.

Die weiteren verwendeten Kurzbezeichnungen und Abkürzungen haben die folgenden Bedeutungen:

| | |
|---|---|
| abs. | absolut |
| Boc | tert-Butoxycarbonyl |
| BOP | Benzotriazol-1-yl-oxy-tris-(di-methyl-amino)-phosponium-hexafluor-phosphat |
| DC | Dünnschichtchromatographie |
| DCC | Dicyclohexylcarbodiimid |
| DEPC | Pyrocarbonsäurediethylester |
| DMF | Dimethylformamid |
| DMSO | Dimethylsulfoxid |
| Essigester | Essigsäureethylester |
| Ether | Diethylether |
| FAB-MS | Fast-Atom-Bombardment-Massenspektroskopie |
| ges. | gesättigt |
| h | Stunde(n) |
| HOBt | 1-Hydroxy-benztriazol |
| IR | Infrarotspektroskopie |
| min | Minute(n) |
| org. | organisch |
| Pd/C | Palladium auf Aktivkohle (Katalysator) |
| RT | Raumtemperatur |
| Smp. | Schmelzpunkt |
| Sole | gesättigte Kochsalzlösung |
| TBAF | Tetrabutylammoniumfluorid (Trihydrat) |
| TFA | Trifluoressigsäure |
| THF | Tetrahydrofuran |
| Z | Benzyloxycarbonyl |

Massenspektroskopische Messwerte werden nach der "Fast-Atom-Bombardment" (FAB-MS)-Methode erhalten. Die Massenangaben beziehen sich auf das protonierte Molekülion $(M+H)^+$.

Die Werte für IR-Spektren werden in $cm^{-1}$ angegeben, in runden Klammern findet sich das jeweilige Lösungsmittel.

Zur Bezeichnung von bivalenten Radikalen von natürlichen α-Aminosäuren werden die in der Peptidchemie üblichen Abkürzungen verwendet. Die Konfiguration am α-Kohlenstoffatom wird durch Voranstellen von (L)- oder (D)- angegeben.

HPLC-Gradienten:

| | |
|---|---|
| I | 20 % → 100 % a) in b) während 20 min. |
| II | 20 % → 100 % a) in b) während 35 min. |
| III | 20 % → 100% a) in b) während 20 min + 100 % a) 8 min. |

Laufmittel a): Acetonitril + 0,05 % TFA; Laufmittel b): Wasser + 0,05 % TFA. Säule (250 x 4,6 mm) gefüllt mit "Reversed-Phase"-Material $C_{18}$-Nucleosil® (5 μm mittlere Korngrösse, mit Octadecylsilanen kovalent derivatisiertes Silicagel, Macherey & Nagel, Düren, BRD). Detektion durch UV-Absorption bei 215 nm. Die Retentionszeiten ($t_{Ret}$) werden in Minuten angegeben. Fliessgeschwindigkeit 1 ml/min.

Der Rest mit der Kurzbezeichnung -Phe[C]Phe- bedeutet das bivalente Radikal von 5(S)-Amino-2(R)-benzyl-4(S)-hydroxy-6-phenyl-hexansäure und hat die Formel

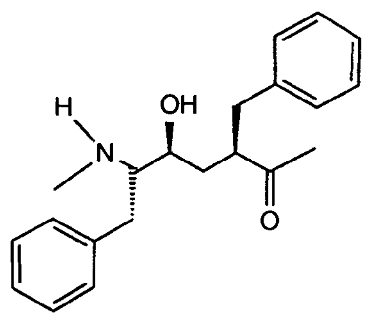

.

Analog bedeutet der Rest mit der Bezeichnung -Phe[C](p-$CH_3O$)Phe- das bivalente Radikal von 5(S)-Amino-4(S)-hydroxy-2(R)-4-methoxybenzyl-6-phenyl-hexansäure, worin in der zuletzt gezeigten Formel anstelle des 2-Phenylmethylrestes ein 2-(4-Methoxyphenyl)methylrest vorliegt.

**Beispiel 1: Boc-Phe[C]Phe-(L)-Val-(L)-Phe-morpholin-4-ylamid**

Eine Lösung von 330,3 mg 5(S)-Boc-amino-4(S)-(tert-butyldimethylsilyloxy)-6-phenyl-2(R)-phenylmethyl-hexanoyl-(L)-Val-(L)-Phe-morpholin-4-ylamid in 3 ml abs. DMF wird mit 247,2 mg TBAF versetzt und das Reaktionsgemisch danach 4,5 h bei RT gerührt. Die farblose Lösung wird auf 50 ml Wasser gegossen und viermal mit Essigester extrahiert. Die vereinten Extrakte werden jeweils mit zweimal 25 ml Natriumbicarbonat-Lösung, zweimal mit Wasser und einmal mit Sole gewaschen und anschliessend über Natriumsulfat getrocknet. Nach Eindampfen des Lösungsmittels wird der Rückstand aus Hexan kristallisiert und die Titelverbindung erhalten. DC $R_f$ (B)= 0,5; FAB-MS $(M+H)^+$= 729.

Das Ausgangsmaterial wird folgendermassen hergestellt:

**1 a) N-3(S)-(Boc-amino)-2(R,S)-hydroxy-4-phenyl-1-trimethylsilyl-butan**

24,7 g Magnesium werden in 100 ml abs. Ether vorgelegt und über 35 min mit wenig Iod und gleichzeitig mit 132,5 ml Chlormethyltrimethylsilan und 300 ml Ether versetzt, wobei die Temperatur mittels Eisbad bei 38 °C gehalten wird. Das erhaltene Reaktionsgemisch wird dann 1,5 h bei RT gerührt. Nach Abkühlen auf -60 °C wird mit einer Suspension von 48,6 g N-Boc-phenylalaninal (Herstellung: D. J. Kempf, J. Org. Chem. 51, 3921 (1986)) in 1,1 l Ether innerhalb 40 min versetzt. Über 90 min wird das Reaktionsgemisch auf RT erhitzt und weitere 90 min bei dieser Temperatur gerührt. Danach wird auf 2 l Eiswasser und 1,5 l 10 %-ige wässrige Zitronensäure gegossen. Die abgetrennte wässrige Phase wird zweimal mit 500 ml Ether extrahiert. Alle Etherextrakte werden mit 500 ml einer 10 %-igen Zitronensäure-Lösung und zweimal mit Sole gewaschen. Nach Trocknen über Natriumsulfat wird unter Vakuum eingeengt und die erhaltene Titelverbindung ohne zusätzliche Reinigung weiterverwendet. DC $R_f$ (C)= 0,6; FAB-MS $(M+H)^+$= 338.

**1 b) 1-Phenyl-3-buten-2(S)-amin**

Eine Lösung von 18,8 g n-3(S)-(Boc-amino)-2-(R,S)-hydroxy-4-phenyl-1-trimethylsilyl-butan in 420 ml Methylenchlorid wird bei 5 °C innerhalb von 10 min mit 35,6 ml einer ungefähr 48 %-igen Lösung von Bortrifluoridethyletherat versetzt. Das Reaktionsgemisch wird dann 16 h bei RT gerührt, auf 10 °C gekühlt und innerhalb von 20 min mit 276 ml einer 4 N Natriumhydroxyd-Lösung versetzt. Die wässrige Phase wird abgetrennt und zweimal mit je 400 ml Methylenchlorid extrahiert. Die vereinigten organischen Extrakte werden mit Sole gewaschen und über Natriumsulfat getrocknet. Das Titelprodukt wird ohne zusätzliche Reinigung weiterverwendet. DC $R_f$ (G)= 0,15 ; IR (Methylenchlorid) ($cm^{-1}$): 3370, 3020, 2920, 1640, 1605.

**1 c) N-Boc-1-phenyl-3-buten-2(S)-amin**

21,5 g 1-Phenyl-3-buten-2(S)-amin werden in 500 ml abs. Methylenchlorid gelöst und tropfenweise mit einer Lösung von 38,3 g Boc-Anhydrid in 250 ml Methylenchlorid versetzt. Nach 1,5 h Rühren bei RT wird auf 100 ml eingeengt, dann mit 1,5 l Ether verdünnt und nacheinander zweimal mit 400 ml 10 %-iger Zitronensäure, einmal mit 400 ml Wasser, einmal mit 400 ml gesättigter wässriger Natriumbicarbonat-Lösung und zweimal mit Sole gewaschen und über Natriumsulfat getrocknet. Nach Eindampfen des Lösungsmittels wird durch Säulenchromatographie gereinigt ($SiO_2$, Hexan/Essigester: 95/5 bis 80/20) und die Titelverbindung aus Hexan auskristallisiert. Smp. 67-68 °C; DC $R_f$ (C)= 0,4; FAB-MS $(M+H)^+$= 248.

**1 d) 2(R)-[1(S)-(Boc-amino)-2-phenylethyl]-oxiran**

Eine Lösung von 1,45 g N-Boc-1-phenyl-3-buten-2(S)-amin in 20 ml Methylenchlorid wird innerhalb von 15 min bei 0 - 5 °C mit einer Lösung von 9,74 g m-Chlorperbenzoesäure in 50 ml Methylenchlorid versetzt. Nach 18 h Rühren bei gleicher Temperatur wird noch weitere 8 h unter Erwärmung auf RT ausgerührt und auf eiskalte 10 %-ige Natriumcarbonat-Lösung gegossen. Die wässrige Phase wird dreimal mit Ether extrahiert. Die vereinigten organischen Phasen werden nacheinander dreimal mit 10 %-iger Natriumsulfit-Lösung, dreimal mit gesättigter Natriumbicarbonat-Lösung, mit Natriumthiosulfat-Lösung und Sole gewaschen und über Natriumsulfat getrocknet. Nach Einengen der Lösungsmittel wird durch Säulenchromatographie ($SiO_2$, Hexan/Essigester: 4/1) die Titelverbindung gereinigt und aus Hexan auskristallisiert. Smp. 51-52 °C; DC $R_f$ (C)= 0,33; FAB-MS $(M+H)^+$= 264.

**1 e) 5(S)-[1(S)-(Boc-amino)-2-phenylethyl]-3(R,S)-carbethoxy-dihydro- furan-2-(3H)-on**

Eine Lösung von 26 ml Malonsäure-diethylester in 260 ml abs. Ethanol wird portionsweise mit 3,4 g Natrium versetzt. Nach Verbrauch des Natriums (ca. 1,5 h) wird innerhalb von 10 min eine Lösung von 13 g 2(R)-[1(S)-(Boc-amino)-2-phenylethyl]-oxiran in 100 ml Ethanol zugetropft. Nach 5 h Rühren bei RT wird das Reaktionsgemisch auf 1,5 l Eiswasser gegossen und mit 10 %-iger Zitronensäure auf pH 4 gestellt. Nach viermaligem Extrahieren mit Ether werden die vereinigten organischen Phasen nacheinander zweimal mit gesättigter wässriger Natriumbicarbonat-Lösung, einmal mit Sole, erneut mit gesättigter wässriger Natriumbicarbonat-Lösung, mit Wasser und nochmals mit Sole gewaschen. Nach Einengen des Lösungsmittels wird durch Säulenchromatographie ($SiO_2$, Hexan/Essigester: 4/1) die Titelverbindung erhalten. DC $R_f$ (C)= 0,22; FAB-MS $(M+H)^+$= 378.

**1 f) 5(S)-[(1(S)-(Boc-amino)-2-phenylethyl]-3(R,S)-carbethoxy-3-phenylmethyl-dihydrofuran-2-(3H)-on**

Eine Lösung von 23,8 g 5(S)-[1(S)-(Boc-amino)-2-phenylethyl]-3(R,S)-carbethoxy-dihydrofuran-2-(3H)-on in 410 ml abs. Ethanol und 14,4 ml Benzylbromid wird zu einer Lösung von 2,76 g Natrium in 410 ml abs. Ethanol gegeben. Das Reaktionsgemisch wird bei RT unter Argon 18 h gerührt und anschliessend auf ein Gemisch von Eis und 10 %-iger Zitronensäure gegossen. Nach dreimaligem Extrahieren mit Ether werden die vereinigten organischen Extrakte mit Wasser und Sole gewaschen und über Natriumsulfat getrocknet. Nach Einengen erhält man die Titelverbindung als farbloses Oel, das ohne zusätzliche Reinigung in die nächste Stufe eingesetzt wird. DC $R_f$ (C)= 0,4; FAB-MS $(M+H)^+$= 468.

**1 g) 5(S)-[1(S)-(Boc-amino)-2-phenylethyl]-3(R)-phenylmethyl-dihydrofuran-2-(3H)-on und 5(S)-[1(S)-(Boc-amino)-2-phenylethyl]-3(S)-phenylmethyl-dihydrofuran-2-(3H)-on**

Eine Lösung von 10 g 5(S)-[(1(S)-(Boc-amino)-2-phenylethyl]-3(R,S)-carbethoxy-3-phenylmethyl-dihydrofuran-2-(3H)-on in 175 ml Dimethoxyethan wird über 5 min tropfenweise mit 81,4 ml einer 1 M wässrigen Lithiumhydroxyd-Lösung bei RT versetzt. Danach wird 15 h bei RT gerührt, und nach Eindampfen des Lösungsmittels wird der erhaltene Rückstand auf 500 ml 10 %-ige Zitronensäure gegossen und dreimal mit Ether extrahiert. Die vereinigten Ether-Phasen werden einmal mit Sole gewaschen und über Natriumsulfat getrocknet. Nach Eindampfen des Lösungsmittels erhält man 9,8 g der rohen Carbonsäure, die durch 14-stündiges Heizen auf 90 °C in 450 ml Toluol zum Titelprodukt decarboxyliert wird. Dieses wird durch Säulenchromatographie (Hexan/Essigester: 9/1) gereinigt, wobei zuerst der 5(S)-[1(S)-(Boc-amino)-2-phenylethyl]-3(R)-phenylmethyl-dihydrofuran-2-(3H)on [DC $R_f$ (C)= 0,3; FAB-MS $(M+H)^+$= 396] und danach der 5(S)-[1(S)-(Boc-amino)-2-phenylethyl]-3(S)-phenylmethyl-dihydrofuran-2-(3H)on [DC $R_f$ (C)= 0,25; FAB-MS $(M+H)^+$= 396] erhalten wird.

[Alternative Synthese für 5(S)-[1(S)-Boc-amino)-2-phenylethyl]-3(R)-phenylmethyl-dihydrofuran-2-(3H)-on), welche auch für grössere Mengen geeignet ist:

$\alpha$) 5(S)-[1(S)-(Boc-amino)-2-phenylethyl]-dihydrofuran-2-(3H)-on

(A.E. DeCamp, A.T. Kawaguchi, R.P. Volante, and I. Shinkai, Tetrahedron Lett. 32, 1867 (1991)). Unter $N_2$-Atmosphäre gibt man zu einer Lösung von 375 g (1,65 Mol) 3-Iodpropionsäureethylester [Herstellung: Eine Suspension von 170 ml 2-Brompropionsäureethylester (Fluka; Buchs, Schweiz) und 950 g Natriumjodid in 1,8 l Aceton wird filtriert, das Filtrat teilweise eingedampft, auf ca. 2,5 l Ether gegossen, mit 1,0 l 1%-iger Natriumthiosulfatlösung und schliesslich Sole gewaschen, mit Natriumsulfat getrocknet und eingedampft. Destillation (83 °C; 20 mbar) liefert den reinen 3-Iodpropionsäureethylester: MS $(M)^+$= 228; $^1$H-NMR (200 MHz, $CDCl_3$): 4,17 (q, 7 Hz, 2 H); 3,34 und 2,97 (2t, 7 Hz, 2 x 2 H); 1,28 (t, 7 Hz, 3 H)] in 1700 ml Toluol 173 g Zn/Cu (Herstellung: R.D. Smith, H.E. Simmons, W.E. Parham, M.D. Bhavsar, Org. Synth., Coll. Vol 5, 855 (1973)) und 280 ml Dimethylacetamid und rührt kräftig während 1 h bei RT und 4 h bei 80°C nach ($\rightarrow$ Zn-Homoenolatlösung). In einer zweiten Apparatur ($N_2$-Atmosphäre) wird eine Lösung von 122 ml (0,40 Mol) Tetraisopropyl-orthotitanat in 350 ml Toluol und 1900

ml Methylenchlorid unter leichtem Kühlen bei einer Innentemperatur von 15 bis 25°C mit 127 ml (1,14 Mol) Titan-tetrachlorid versetzt, 15 min bei RT gerührt (→ gelbe Lösung) und auf -40°C abgekühlt (→ teilweise Kristallisation des Trichlor-titan-isopropylats). Die auf RT abgekühlte Zn-Homoenolatlösung filtriert man unter Argonatmosphäre durch eine G3-Glasfritte und tropft sie zum Trichlor-titan-isopropylat, wobei die Temperatur bei -30°C bis -25°C gehalten wird (→ tiefrote Lösung), rührt während 5 min bei -25°C und kühlt auf -40°C ab. Anschliessend tropft man eine Lösung von 233 g (0,85 Mol) N-Boc-phenylalaninal (Herstellung: D.J. Kempf, J. Org. Chem. 51, 3921 (1986), dann Kristallisation aus Hexan (0°C, ca. 18 h), Waschen mit kaltem Hexan, Trocknen) in 1500 ml Methylenchlorid zu und rührt 15 h bei -22 bis -18°C und schliesslich 1 h bei 0°C nach. Das Reaktionsgemisch wird in 10 l Eiswasser und 12 l tert-Butylmethylether aufgenommen und 7-10 min kräftig gerührt. Man trennt die wässrige Phase ab; extrahiert sie 2x mit 10 l Ether; wäscht die organischen Phasen mit 8 l Wasser, 8 l ges. Natriumhydrogencarbonat-Lösung, 8 l Wasser und 5 l Sole; trocknet sie mit Natriumsulfat und dampft sie ein (→ kristalliner 5(S)-(Boc-amino)-4(S)-hydroxy-6-phenyl-hexansäure-ethylester). Das obige Zwischenprodukt wird in 6500 ml Toluol und 230 ml Essigsäure unter Argonatmosphäre während 2,5 h auf 100°C erhitzt. Man giesst das erkaltete Reaktionsgemisch unter Rühren auf 6 l Eiswasser, trennt die wässrige Phase ab, extrahiert sie 2x mit 2000 ml Toluol, wäscht die org. Phasen mit 5 l ges. Natriumhydrogencarbonat Lösung, 5 l 40 %-iger Natriumhydrogensulfit Lösung, 4 l Wasser und 4 l Sole und trocknet sie mit Natriumsulfat. Eindampfen der org. Phasen bis zu einem Rückstand von ca. 300 g und Versetzen mit 800 ml Hexan (mehrere Stunden ausrühren) liefert kristallines Lacton, das laut HPLC ca. 10 % des (5R)-Epimeren (DC $R_f$(E)=0,08; $t_{Ret}$(II)=18,8 min) enthält. Dieses Material wird in der nächsten Stufe eingesetzt. Die reine Titelverbindung kann nach Säulenchromatographie ($SiO_2$, Hexan/Essigsäureethylester 2:1) erhalten werden: DC $R_f$(E)=0,14; $t_{Ret}$(II)=19,2.

β) <u>5(S)-[1(S)-(Boc-amino)-2-phenylethyl]-3(R)-phenylmethyl-dihydrofuran-2-(3H)-on</u>

(A.K. Ghosh, S.P. McKee, and W.J. Thompson, J. Org. Chem. 56, 6500 (1991)). Unter $N_2$-Atmosphäre wird eine Lösung von 1943 g (6,32 Mol) 5(S)-[1(S)-(Boc-amino)-2-phenylethyl]-dihydrofuran-2-(3H)-on in 12,0 l THF und 1,9 l 1,3-Dimethyl-3,4,5,6-tetrahydro-2(1H)-pyrimidinon auf -75°C abgekühlt, bei einer Innentemperatur von unter -70°C mit 14000 ml Lithiumbis(trimethylsilyl)amid (1 M) in THF (Aldrich) versetzt und 20 min bei -75°C nachgerührt. Dazu tropft man während 1 h 835 ml (7,00 Mol) Benzylbromid, wobei die Innentemperatur -70°C nicht übersteigen darf, und rührt während 30 min bei -75°C aus. Zur klaren Lösung gibt man anschliessend bei -75 bis -70°C 2320 ml Propionsäure (90 min) und dann 2320 ml Wasser (1 h), wobei man die Temperatur auf -10°C ansteigen lässt. Das Reaktionsgemisch wird auf 30 l Essigsäureethylester und 35 l 10 %-ige Zitronensäure-Lösung gegossen, die wässrige Phase abgetrennt und mit 2x 10l Essigsäureethylester nachextrahiert. Die organischen Phasen wäscht man mit 3x 12 l ges. Natriumbicarbonat Lösung, 20 l Sole und 2x 20 l Wasser, engt sie ein, nimmt den öligen Rückstand in 10 l Toluol auf und dampft bis zu einem Restvolumen von ca. 5 l ein. Filtrieren des Eindampfrückstandes durch 4 kg Kieselgel Merck (0,063-0,200 mm), Nachwaschen mit Toluol und Kristallisieren des Rohprodukts aus Hexan (4 l Hexan/kg Rohprodukt) liefert die Titelverbindung: DC $R_f$(D)=0,54; FAB-MS $(M+H)^+$=414.]

**1 h) <u>5(S)-(Boc-amino)-4(S)-hydroxy-6-phenyl-2(R)-phenylmethyl-hexansäure</u>**

Eine Lösung von 17,6 g 5(S)-[1(S)-(Boc-amino)-2-phenylethyl]-3(R)-phenylmethyl-dihydrofuran-2-(3H)on in 710 ml Ethylenglykoldimethylether und 352 ml Wasser wird tropfenweise innerhalb von 10 min mit 176 ml einer 1 M Lithiumhydroxyd-Lösung bei 20 °C versetzt. Danach wird das Reaktionsgemisch 1,5 h bei RT gerührt und das Lösungsmittel eingedampft. Der Rückstand wird auf 1 l kalte 10 %-ige Zitronensäure gegossen und die saure Lösung dreimal mit je 800 ml Essigester extrahiert. Die vereinten Extrakte werden erst mit 800 ml Wasser, dann mit 800 ml Sole gewaschen. Nach Trocknen der organischen Lösung über Natriumsulfat wird das Lösungsmittel abdestilliert. Die rohe Titelverbindung wird ohne weitere Reinigung in die nächste Stufe eingesetzt. FAB-MS $(M+H)^+$= 414.

**1 i) <u>5(S)-(Boc-amino)-4(S)-(tert-butyldimethylsilyloxy)-6-phenyl-2(R)-phenyl-methyl-hexansäure</u>**

Eine Lösung von 6,35 g 5(S)-(Boc-amino)-4(S)-hydroxy-6-phenyl-2(R)-phenylmethyl-hexansäure in 90 ml DMF wird unter Rühren mit 8 g Imidazol und 10 g t-Butyldimethylchlorsilan versetzt. Nach 18 h Rühren bei RT wird die gelbe klare Lösung auf Eiswasser gegossen und dreimal mit je 250 ml Essigester extrahiert. Die vereinten Extrakte werden nacheinander dreimal mit 10 %-iger Zitronensäure, einmal mit Wasser, dreimal mit wässriger gesättigter Natriumbicarbonat-Lösung, einmal mit Wasser und zuletzt mit Sole gewaschen. Nach Trocknen über Natriumsulfat wird das Lösungsmittel eingedampft und der so erhaltene tert-Butyldimethylsilyl-ether (13,5 g) in 53 ml THF gelöst und mit 53 ml Essigsäure und 20 ml Wasser behandelt. Nach 3 h Rühren bei RT wird auf Wasser gegossen und dreimal mit Ether extrahiert. Die gesammelten Ether-Extrakte werden zweimal mit Wasser und einmal mit Sole gewaschen und über Natriumsulfat getrocknet. Nach Einengen wird das Rohprodukt durch Säulenchromatographie ($SiO_2$, Hexan/Essigester:

3,5/1,5) gereinigt und die Titelverbindung erhalten. DC $R_f$ (D)= 0,37; FAB-MS $(M+H)^+$= 528.

**1 j) 5(S)-(Boc-amino)-4(S)-(tert-butyldimethylsilyloxy)-6-phenyl-2(R)-phenyl-methyl-hexanoyl-(L)-Val-(L)-Phe-morpholin-4-ylamid**

Eine Lösung von 250 mg 5(S)-(Boc-amino)-4(S)-tert-butyldimethylsilyloxy)-6-phenyl-2(R)-phenylmethyl-hexansäure in 3 ml DMF mit 230,5 mg BOP, 70,4 mg HOBT und 182,6 ml N-Methylmorpholin wird 30 min bei RT in etwa 2 ml DMF gerührt und dann mit 189,5 mg H-(L)-Val-(L)-Phe-morpholin-4-yl-amid [Herstellung siehe unter 1k) bis 1n)] versetzt. Nach 16 h bei RT dampft man ein und verteilt den Rückstand zwischen 3 Portionen Essigester, Wasser, ges. Natriumbicarbonat-Lösung, Wasser und Sole, trocknet die organischen Phasen mit Natriumsulfat und dampft sie ein. Man erhält die Titelverbindung als Rohprodukt; DC $R_f$ (A)= 0,24; FAB-MS $(M+H)^+$= 843.

**1 k) Z-(L)-Phe-morpholin-4-ylamid**

Eine Lösung von 4,49 g Z-(L)-Phe-OH in 190 ml Methylenchlorid wird auf 0 °C gekühlt und mit 3,09 g DCC versetzt. Nach 20 min Rühren bei 0 °C wird über 15 min eine Lösung von 1,31 ml Morpholin in 10 ml Methylenchlorid zugetropft. Das Reaktionsgemisch wird weitere 24 h bei RT gerührt, und nach Abfiltrieren des ausgefallenen Dicyclohexylharnstoffes wird nacheinander mit Methylenchlorid, wässriger Natriumbicarbonat-Lösung und Sole gewaschen. Nach Trocknen über Natriumsulfat und Einengen erhält man die rohe Titelverbindung, die aus Ether auskristallisiert. DC $R_f$(B)= 0,55.

**1 l) H-(L)-Phe-morpholin-4-ylamid**

Eine Lösung von 5,5 g Z-(L)-Phe-morpholin-4-ylamid mit 1,5 g 10 %-igem Pd/C in 150 ml Methanol wird bei RT während 1 h mit der berechneten Menge Wasserstoff durch Hydrogenolyse in die Titelverbindung überführt. Nach Abfiltrieren des Katalysators wird eingeengt, und nach Verdünnen mit Essigester wird die erhaltene Lösung mit einer gesättigten Natriumbicarbonat-Lösung gewaschen, über Natriumsulfat getrocknet und unter vermindertem Druck eingeengt. Nach Säulenchromatographie (analog Beispiel 1 n)) wird die Titelverbindung in reiner Form erhalten. DC $R_f$(F)= 0,3.

**1 m) Z-(L)-Val-(L)-Phe-morpholin-4-ylamid**

Eine Lösung von 2,14 g Z-(L)-Val-OH in 80 ml abs. eisgekühltem Methylenchlorid wird mit 1,75 g DCC versetzt und nach 20 min Rühren bei dieser Temperatur tropfenweise über 15 min mit einer Lösung von 2 g H-(L)-Phe-morpholin-4-ylamid versetzt. Das Reaktionsgemisch wird weitere 24 h bei RT gerührt, und der entstandene Harnstoff wird abfiltriert. Das Filtrat wird nacheinander mit wässriger Natriumbicarbonat-Lösung und Sole gewaschen und nach Trocknen über Natriumsulfat eingeengt. Durch Verrühren mit Ether und Abfiltrieren des unlöslichen Rückstandes erhält man nach Einengen die Titelverbindung, die ohne zusätzliche Reinigung weiterverarbeitet wird. DC $R_f$(F)= 0,7.

**1 n) H-(L)-Val-(L)-Phe-morpholin-4-ylamid**

Analog Beispiel 1 l) werden 3,9 g Z-(L)-Val-(L)-Phe-morpholin-4-ylamid durch Hydrogenolyse über 0,5 g 10 %-igem Pd/C in 150 ml Methanol in die rohe Titelverbindung überführt, die durch Säulenchromatographie ($SiO_2$, Methylenchlorid bis Methylenchlorid/Methanol: 97,5 zu 2,5 (v/v)) gereinigt wird. DC $R_f$(F)= 0,4.

**Beispiel 2: Boc-Phe[C](p-$CH_3O$)Phe-(L)-Val-(L)-Phe-morpholin-4-ylamid**

Analog Bsp. 1) werden 417 mg (0,48 mMol) 5(S)-(Boc-amino)-4(S)-(tert-butyldimethylsilyloxy)-6-phenyl-2(R)-(p-methoxy-phenylmethyl)-hexanoyl-(L)-Val-(L)-Phe-morpholin-4-ylamid mit 301 mg (0,95 mMol) TBAF in 5 ml DMF zur Titelverbindung entschützt: DC $R_f$(F)=0,4; $t_{Ret}$(I)=15,8 min; FAB-MS $(M+H)^+$=759.

Das Ausgangsmaterial wird wie folgt hergestellt:

**a) p-Methoxy-benzyljodid**

Eine Lösung von 1,7 ml (12,8 mMol) 4-Methoxy-benzylchlorid (Fluka; Buchs/Schweiz) in 25 ml Aceton wird mit 9,4 g (62,6 mMol) Natriumjodid bei RT gerührt. Ein Gaschromatogramm des Reaktionsgemisches nach 90 min zeigt vollständigen Umsatz an, deshalb giesst man das Reaktionsgemisch auf Ether und wäscht es mit 10-%iger Natriumthiosulfat Lösung und Sole. Trocknen der organischen Phase mit $Na_2SO_4$ und Eindampfen liefert die Titelverbindung: $^1$H-NMR (200 MHz, $CD_3OD$: 3,78 (s, 3 H), 4,54 (s, 2 H), 6,8-6,95 und 7,2-7,4 (2m, je 2 H).

**b) 5(S)-[1(S)-(Boc-amino)-2-phenyl-ethyl]-3(R)-(p-methoxy-phenylmethyl)-dihydro-furan-2-(3H)-on**

Es werden 2,98 g (9,74 mMol) 5(S)-[1(S)-(Boc-amino)-2-phenylethyl]-dihydrofuran-2-(3H)-on gelöst in 40 ml THF bei -75°C mit 19,5 ml Lithium-bis(trimethylsilyl)amid 1 M in THF deprotoniert und mit 2,9 g (11,7 mMol) p-Methoxy-benzyljodid in 20 ml THF alkyliert (45 min). Säulenchromatographie ($SiO_2$, Hexan/Essigsäureethylester 2:1) und Digerieren aus Diisopropylether liefert die reine Titelverbindung: DC $R_f$(D)=0,32; $t_{Ret}$(I)=16,7 min.

**c) 5(S)-(Boc-amino)-4(S)-hydroxy-6-phenyl-2(R)-(p-methoxy-phenylmethyl)-hexansäure**

Es werden 1,7 g (3,99 mMol) 5(S)-[1(S)-(Boc-amino)-2-phenylethyl]-3(R)-(p-methoxyphenylmethyl)-dihydrofuran-2-(3H)-on in 43 ml Dimethoxyethan und 11 ml Wasser mit 16 ml 1 M Lithiumhydroxid Lösung hydrolysiert. Verrühren in Ether liefert die reine Titelverbindung: DC $R_f$(F)=0,53; $t_{Ret}$(I)=14,2 min; FAB-MS $(M+Na)^+$=466.

d) **5(S)-(Boc-amino)-4(S)-(tert-butyldimethylsilyloxy)-6-phenyl-2(R)-(p-methoxy-phenylmethyl)-hexansäure**

Analog Bsp. 1i) werden 0,93 g (2,10 mMol) 5(S)-(Boc-amino)-4(S)-hydroxy-6-phenyl-2(R)-(p-methoxy-phenylmethyl)-hexansäure in 20 ml DMF mit 1,4 g (9,64 mMol) tert-Butyldimethylchlorsilan und 1,17 g (17,2 mMol) Imidazol silyliert. Hydrolyse der Silylesterfunktion mit 1,7 g Kaliumcarbonat in Methanol (23 ml)/THF (7 ml)/Wasser (7 ml) und Verrühren des Rohproduktes in Hexan liefert die Titelverbindung: $t_{Ret}$(I)=20,6 min; FAB-MS $(M+H)^+$=558.

e) **5(S)-(Boc-amino)-4(S)-(tert-butyldimethylsilyloxy)-6-phenyl-2(R)-(p-methoxy-phenylmethyl)-hexanoyl-(L)-Val-(L)-Phe-morpholin-4-ylamid**

Es werden 300 mg (0,537 mMol) 5(S)-(Boc-amino)-4(S)-(tert-butyldimethylsilyloxy)-6-phenyl-2(R)-(p-methoxy-phenylmethyl)-hexansäure und 197 mg (0,59 mMol) H-(L)-Val-(L)-Phe-morpholin-4-ylamid (Bsp. 1n) in 5,2 ml NMM/$CH_3CN$ 0,25 M mit 224 mg (0,59 mMol) HBTU umgesetzt: $t_{Ret}$(I)=22,1 min; FAB-MS $(M+H)^+$=873.

f) **5(S)-[1(S)-(Boc-amino)-2-phenylethyl]-dihydrofuran-2-(3H)-on**

(Analog A. E. DeCamp et al., Tetrahedron Lett. 32, 1867 (1991).) Unter $N_2$-Atmosphäre gibt man zu einer Lösung von 17,4 g 2-Iodpropionsäureethylester in 130 ml Toluol 8,03 g Zn/Cu (Herstellung: siehe R.D. Smith, H.E. Simmons, W.E. Parham, M.D. Bhasvar, Org. Synth., Coll. Vol 5, 855 (1973)) und 12,96 ml Dimethylacetamid und rührt kräftig während 1 h bei RT und 4 h bei 80 °C nach (unter Erhalten der entsprechenden Zn-Homoenolat-Lösung). In einer zweiten Apparatur ($N_2$-Atmosphäre) wird eine Lösung von 5,58 ml (18,9 mMol) Tetraisopropy-orthotitanat in 1,64 ml Toluol und 91,8 ml Methylenchlorid unter leichtem Kühlen mit 5,90 ml (53,8 mMol) Titan-tetrachlorid versetzt, 15 min bei RT gerührt (ergibt gelbe Lösung) und auf -40 °C abgekühlt (es erfolgt teilweise Kristallisation des Trichlor-titan-isopropylats). Mittels Kanüle dekantiert man die auf RT abgekühlte Zn-Homoenolat-Lösung vom metallischen Festkörper und tropft sie zum Trichlor-titanisopropylat, wobei die Temperatur bei -40 °C bis -30 °C gehalten wird (ergibt eine tiefrote Lösung), erwärmt während 5 min auf -25 °C und kühlt wiederum auf -40 °C ab. Anschliessend tropft man eine Lösung von 9,0 g N-Boc-phenylalaninal (Herstellung: D.J. Kempf, J. Org. Chem 51, 3921 (1986)) in 32,8 ml Methylenchlorid zu und rührt 15 h bei ca -20 °C und schliesslich bei 0 °C nach. Das Reaktionsgemisch wird auf 0,5 kg Eiswasser und 0,5 l Diethylether gegossen und 10 min kräftig gerührt. Man trennt die wässrige Phase ab, extrahiert sie mit 2 Portionen Diethylether; wäscht die organischen Phasen mit 2 Portionen Wasser, gesättigter Natriumhydrogencarbonat-Lösung und Sole, trocknet sie mit Natriumsulfat und dampft sie ein. Man erhält als Zwischenprodukt kristallinen 5(S)-(Boc-amino)-4(S)-hydroxy-6-phenyl-hexansäureethylester. Dieses Zwischenprodukt wird in 195 ml Toluol und 9 ml Essigsäure während 2,5 h auf 80 °C erhitzt. Man versetzt das Reaktionsgemisch mit 0,5 ml Wasser, trennt die organische Phase ab, extrahiert sie mit 2 Portionen Diethylether, wäscht die organischen Phasen mit gesättigter Natriumhydrogencarbonat-Lösung, Wasser und Sole und trocknet sie mit Natriumsulfat. Teilweises Eindampfen der organischen Phasen und Versetzen mit Hexan liefert die kristalline Titelverbindung, die laut Analytik ca 10% des 4(R)-Epimeren (DC $R_f$(E)=0,08) enthält. Säulenchromatographie ($SiO_2$, Hexan/Essigsäureethylester) liefert die reine Titelverbindung: DC ($R_f$(E)=0,14; $[\alpha]^D$=17,7 (c=1; Ethanol).

**Beispiel 3: 5(S)-(Boc-amino)-4(S)-hydroxy-6-cyclohexyl-2(R)-(4-methoxy-phenyl-methyl)-hexanoyl-(L)-Val-(L)-Phe-morpholin-4-ylamid**

Analog Beispiel 1 werden 3,93 g (4,469 mMol) 5(S)-(Boc-amino)-4(S)-(tert-butyldimethylsilyloxy)-6-cyclohexyl-2(R)-(4-methoxyphenyl-methyl)-hexanoyl-(L)-Val-(L)-Phe-morpholin-4-ylamid in 15 ml DMF mit 2,82 g (8,94 mMol) TBAF-Trihydrat zur Titelverbindung umgesetzt. Die Titelverbindung wird durch Fällung (Hexan) gereinigt. DC $R_f$ (B)= 0,64; $t_{Ret}$(III)= 17,34 min; FAB-MS $(M+H^+)$= 765.

Die Ausgangsverbindung wird folgendermassen hergestellt:

a) **5(S)-(Boc-amino)-4(S)-(tert-butyldimethyl)silyloxy-6-cyclohexyl-2(R)-(4-methoxy-phenyl-methyl)-hexanoyl-(L)-Val-(L)-Phe-morpholin-4-ylamid**

Eine Lösung von 4 g (4,623 mMol) 5(S)-(Boc-amino)-4(S)-(tert-butyldimethyl)silyloxy-6-cyclohexyl-2(R)-(4-hydroxyphenyl-methyl)-hexanoyl-(L)-Val-(L)-Phe-morpholin-4-ylamid in 70 ml Dioxan mit 6,02 g (18,49 mMol) Caesiumcarbonat und 9,1 ml (92,46 mMol) Methyljodid behandelt und anschliessend auf 50 °C erhitzt. Nach 1,25 h wird nochmals die gleiche Menge Caesiumcarbonat und Methyliodid zugegeben, und nach 2,15 h und 4 h wird jeweils nochmals die gleiche Menge an Methyliodid zugegeben. Nach insgesamt

5,75 h Rühren bei 50 °C wird das Reaktionsgemisch auf Eis/Wasser gegossen und 3 mal mit Methylenchlorid extrahiert. Nach dem Trocknen über Natriumsulfat wird am Rotationsverdampfer eingeengt. Die nach Aufarbeiten erhaltene Titelverbindung wird ohne weitere Reinigung weiterverarbeitet. DC $R_f$ (H)= 0,36; $t_{Ret}$(III)= 24 min; FAB-MS (M+H$^+$)= 880.

**b) 5(S)-(Boc-amino)-4(S)-(tert-butyldimethyl)silyloxy-6-cyclohexyl-2(R)-(4-hydroxyphenyl-methyl)-hexanoyl-(L)-Val-(L)-Phe-morpholin-4-ylamid**

Es werden 19,5 g (20,41 mMol) 5(S)-(Boc-amino)-4(S)-(tert-butyldimethylsilyloxy)-6-cyclohexyl-2(R)-(4-benzyloxyphenyl-methyl)-hexanoyl-(L)-Val-(L)-Phe-morpholin-4-ylamid in 400 ml Methanol in Gegenwart von 4 g 10% Pd/C hydriert. Die nach Aufarbeiten erhaltene Titelverbindung wird ohne zusätzliche Reinigung weiterumgesetzt; DC $R_f$ (A) 0,28; $t_{Ret}$(III) = 21,99 min; FAB-MS (M+H$^+$)= 866.

**c) 5(S)-(Boc-amino)-4(S)-(tert-butyldimethyvl)silyloxy-6-cyclohexyl-2(R)-(4-benzyloxyphenyl-methyl)-hexanoyl-(L)-Val-(L)-Phe-morpholin-4-ylamid**

Es werden 3 g (4,69 mMol) 5(S)-(Boc-amino)-4(S)-(tert-butyldimethylsilyloxy)-6-cyclohexyl-2(R)-(4-benzyloxy-benzyl)-hexansäure in 40 ml DMF mit 1,91 g (5,16 mMol) H-(L)-Val-(L)-Phe-morpholin-4-ylamid im Eisbad auf 5 °C gekühlt und mit 0,783 ml (5,16 mMol) DEPC und 2,3 ml (16,41 mMol) Triethylamin versetzt. Nach 1,5 h Rühren bei RT wird auf Wasser gegossen und 3 mal mit Essigsäureethylester extrahiert. Die vereinigten organischen Phasen werden mit Wasser, gesättigter Natriumbicarbonat Lösung (2 mal) und Sole gewaschen und nach Trocknen über Natriumsulfat unter vermindertem Druck eingeengt. Die Titelverbindung wird durch Säulenchromatographie gereinigt ($SiO_2$, Hexan/Essigsäureethylester: 1/1); DC $R_f$ (A)= 0,3; $t_{Ret}$(III) = 25,3 min; FAB-MS (M+H$^+$)= 955.

**d) 5(S)-(Boc-amino)-4(S)-(tert-butyldimethyl)silyloxy-6-cyclohexyl-2(R)-(4-benzyloxy-benzyl)-hexansäure.**

Analog Beispiel 1 i) werden 28,8 g (54,8 mMol) 5(S)-(Boc-amino)-4(S)-hydroxy-6-cyclohexyl-2(R)-(4-benzyloxy-benzyl)-hexansäure in 288 ml DMF mit 35,8 g (237,6 mMol) tert-Butyldimethylchlorsilan und 30 g (237,6 mMol) Imidazol in die Titelverbindung überführt. Die Titelverbindung wird durch Säulenchromatographie gereinigt ($SiO_2$, Hexan/Essigsäureethylester: 4/1 bis 1/1); DC $R_f$ (E)= 0,33; $t_{Ret}$(III)= 23,72 min;

**e) 5(S)-(Boc-amino)-4(S)-hydroxy-6-cyclohexyl-2(R)-(4-benzyloxy-benzyl)-hexansäure**

Analog Beispiel 1 h) werden 2,4 g (4,728 mMol) 5(S)-[1(S)-(Boc-amino)-2-cyclohexylethyl]-3(R)-(4-benzyloxy-benzyl)-dihydrofuran-2-(3H)-on in 10 ml 1,2-Dimethoxyethan mit 9,45 ml 1M LiOH-Lösung zur Titelverbindung umgesetzt. Diese wird durch Kristallisation aus Hexan gereinigt. DC $R_f$ (E) = 0,33; $t_{Ret}$(III)= 18 min; FAB-MS (M+H$^+$)= 526.

**f) b) 5(S)-[1(S)-(Boc-amino)-2-cyclohexylethyl]-3(R)-(4-benzyloxybenzyl)-dihydrofuran-2-(3H)-on**

(Analog A.K. Ghosh et al., J. Org. Chem. 56, 6500 (1991)) werden 30,9 g (99,26 mMol) 5(S)-[1(S)-(Boc-amino)-2-cyclohexylethyl]-dihydrofuran-2-(3H)-on mit 200 ml (200 mMol) Lithium-bis(trimethylsilyl)amid (Aldrich) 1M in THF bei -75 °C durch Zutropfen von 34 g (104,8 mMol) 4-Benzyloxybenzyljodid unter 30-minütigem Ausrühren bei -75 °C zur Titelverbindung umgesetzt. Nach erneutem Abkühlen auf -75 °C werden Propionsäure und dann Wasser zugesetzt. Man wärmt auf 0 °C auf, verdünnt mit Essigsäureethylester, wäscht mit 10%-iger Zitronensäurelösung, gesättigter Natriumcarbonat-Lösung und Sole, trocknet über Natriumsulfat und dampft ein. Die Titelverbindung wird durch Säulenchromatographie ($SiO_2$, Hexan/Essigsäureethylester: 4/1 bis 1/1) und Kristallisation (Hexan/Essigsäureethylester) gereinigt; DC $R_f$ (C)= 0,33; $t_{Ret}$(III)= 20,41 min; FAB-MS (M+H$^+$)= 508.

**g) 5(S)-[1(S)-(Boc-amino)-2-cyclohexylethyl]-dihydrofuran-2-(3H)-on**

Eine Lösung von 5 g (16,37 mMol) 5(S)-[1(S)-(Boc-amino)-2-phenylethyl]-dihydrofuran-2-(3H)-on in 50 ml Methanol wird in Gegenwart von 0,5 g Nishimura-Katalysator unter Normaldruck 2 h bei RT hydriert. Nach Abfiltrieren des Katalysators wird am Rotationsverdampfer eingeengt und am Hochvakuum getrocknet. DC $R_f$ (D)= 0,5; FAB-MS (M+H$^+$)= 312.

**Beispiel 4: Hemmung des Wachstums eines humanen Mammacarcinoms im Mausmodell:**

2 x 6 weiblichen Balb/c nu/nu-Mäusen (Bomholtgard, östrogenstimuliert mit 5 mg Östradiol in einem perforierten Plastikschlauch "Silastic®" (Dow Chemical, Michigan, USA)) werden durch subkutane Transplantation von humanen Mammacarcinomstücken (ca. 25 mg) der Linie MCF-7 (American Type Culture Collection, Maryland, USA; vgl. H.D. Soule et al., J. Nat. Cancer Inst. 51, 1409-16 (1973)) Tumoren implantiert. Nach Wachstum der Tumoren auf ein Volumen von etwa 30 bis 100 mm$^3$ (nach 13 Tagen) erhalten 6 Mäuse (Testgruppe) während eines Zeitraums von 25 Tagen zweimal täglich eine Dosis von je 50 mg/kg Boc-Phe[C]Phe-(L)-Val-(L)-Phe-morpholin-4-ylamid (gelöst in einer Konzentration von 2 mg/ml in einer 5 %-igen Lösung von Dimethylsulfoxid und 20 % Hydroxypropyl-β-cyclodextrin in Wasser, hergestellt durch Auflösen der Testverbindung in Dimethylsulfoxid und Verdünnen mit wässriger Hydroxypropyl-β-cyclodextrin-Lösung). Die Applikation erfolgt oral durch

Eingeben der Lösung in den Rachen der Versuchstiere. Die anderen 6 Mäuse (Kontrollgruppe) erhalten parallel Placebo (5 %-ige Lösung von Dimethylsulfoxid in 20 % wässriger Hydroxypropyl-β-cyclodextrin-Lösung). Nach 25 Tagen (50 Applikationen) werden folgende Tumorvolumina (Mittelwert) ermittelt:

Kontrollgruppe (Placebo):

Tumorvolumen 0,96 $cm^3$.

Testgruppe (Boc-Phe[C]Phe-(L)-Val-(L)-Phe-morpholin-4-ylamid):

Tumorvolumen 0,30 $cm^3$.

Es zeigt sich eine deutliche Verringerung des Tumorwachstums bei den mit Boc-Phe[C] Phe-(L)-Val-(L)-Phe-morpholin-4-ylamid behandelten Tieren im Vergleich mit der Kontrollgruppe.

Das Tumorwachtums wird ermittelt durch Messung des Durchmessers entlang der Längsachse des Tumors (L) und senkrecht dazu (D) (am lebenden Tier mittels einer Schiebelehre). Die Tumorvolumina werden berechnet nach der Formel $\pi \times L \times D^2/6$ (Evans, B.D., et al., Brit. J. Cancer 45, 466-8 (1982)).

**Beispiel 5: Gelatine-Lösung**

Eine sterilfiltrierte wässrige Lösung des in Beispiel 1 genannten Wirkstoffes der Formel I, die zusätzlich 20 % Cyclodextrin enthält, und eine sterile, mit Phenol konservierte Gelatinelösung werden unter Erwärmen unter aseptischen Bedingungen so vermischt, dass 1,0 ml Lösung folgender Zusammensetzung resultiert:

| | |
|---|---|
| Wirkstoff | 3 mg |
| Gelatine | 150,0 mg |
| Phenol | 4,7 mg |
| dest. Wasser mit 20 % Cyclodextrinen | 1,0 ml |

**Beispiel 6: Sterile Trockensubstanz zur Injektion**

Man löst 5 mg des in Beispiel 1 genannten Wirkstoffes der Formel I in 1 ml einer wässrigen Lösung mit 20 mg Mannit und 20 % Cyclodextrinen als Lösungsvermittler. Die Lösung wird sterilfiltriert und unter aseptischen Bedingungen in eine 2 ml-Ampulle gefüllt, tiefgekühlt und lyophilisiert. Vor dem Gebrauch wird das Lyophilisat in 1 ml destilliertem Wasser oder 1 ml physiologischer Kochsalzlösung gelöst. Die Lösung wird intramuskulär oder intravenös angewendet. Diese Formulierung kann auch in Doppelkammerspritzampullen abgefüllt werden.

**Beispiel 7: Nasenspray**

In einer Mischung von 3,5 ml Myglyol 812® und 0,08 g Benzylalkohol werden 500 mg fein gemahlenes (<5,0 μm) Pulver des in Beispiel 1 genannten Wirkstoffes der Formel I suspendiert. Diese Suspension wird in einen Behälter mit Dosierventil eingefüllt. Es werden 5,0 g Freon 12® unter Druck durch das Ventil in einen Behälter abgefüllt. Durch Schütteln wird das "Freon" in der Myglyol-Benzylalkoholmischung gelöst. Dieser Spraybehälter enthält ca. 100 Einzeldosen, die einzeln appliziert werden können.

**Beispiel 8: Lacktabletten**

Für die Herstellung von 10 000 Tabletten enthaltend je 100 mg Wirkstoff werden folgende Bestandteile verarbeitet:

| | |
|---|---|
| Wirkstoff | 1000 g |
| Maisstärke | 680 g |
| Kolloidale Kieselsäure | 200 g |
| Magnesiumstearat | 20 g |
| Stearinsäure | 50 g |
| Natriumcarboxymethylstärke | 250 g |
| Wasser | quantum satis |

Ein Gemisch des in Beispiel 1 genannten Wirkstoffes der Formel I, 50 g Maisstärke und kolloidaler Kieselsäure wird mit Stärkekleister aus 250 g Maisstärke und 2,2 kg entmineralisiertem Wasser zu einer feuchten Masse verarbeitet. Diese wird durch ein Sieb von 3 mm Maschenweite getrieben und bei 45 ° während 30 Min. im Wirbelschichttrockner getrocknet. Das getrocknete Granulat wird durch ein Sieb von 1 mm Maschenweite gedrückt, mit einer vorher gesiebten Mischung (1 mm Sieb) von 330 g Maisstärke, des Magnesiumstearats, der Stearinsäure und der Natriumcarboxymethylstärke gemischt und zu schwach gewölbten Tabletten verpresst.

**Beispiel 9: Oral verabreichbare Dispersion 1**

625 mg des in Beispiel 1 genannten Wirkstoffes der Formel I, Boc-Phe[C]Phe-(L)-Val-(L)-Phe-morpholin-4-ylamid, und 625 mg POPC (1-Palmitoyl-2-oleoyl-phosphatidylcholin = 1-Hexadecanoyl-2-(9-cis-octadecenoyl)-3-sn-phosphatidylcholin) werden in 25 ml Ethanol gelöst. Die Lösung wird mit der zehnfachen Menge Wasser verdünnt. Hierzu wird die ethanolische Lösung bei Raumtemperatur mit einer Rate von 10 mVmin zu der vorgelegten Menge Wasser zugetropft. Das Ethanol wird aus der Mischung durch tangentiale Dialyse ("Cross Flow Filtration") gegen 1750 ml Wasser (System: Minitan®, 700 $cm^2$- Polyethersulphon-Membran mit einer Ausschlussgrenze von 100 kD, der Firma Millipore (USA)) entfernt. Die Mischung wird unter Verwendung desselben Systems auf 15 mg Wirkstoff konzentriert durch Ultrafiltration. Nach Zugabe von 1,24 mg/ml Zitronensäure und 1,24 mg/ml Dinatriumhydrogenphosphat·2 $H_2O$ zur Einstellung auf pH 4,2 und von 1 mg/ml Sorbinsäure als antimikrobiellem Konservierungsmittel wird die Dispersion erneut auf 15 mg/ml aufkonzentriert und in Gläschen, beispielsweise mit 20 ml Inhalt, abgefüllt. Die Dispersionspartikel haben einen Durchmesser von 0,1 - 2 μm. Sie sind bei +2 bis 8 °C mindestens ein halbes Jahr lang stabil und geeignet zur oralen Verabreichung.

**Beispiel 10: Oral verabreichbare Dispersion 2**

Die Herstellung erfolgt analog Beispiel 9, jedoch unter Verwendung von 25 mg Wirkstoff und 50 mg POPC zur Herstellung der ethanolischen Lösung.

**Beispiel 11: Oral verabreichbare Dispersion 3**

Die Herstellung erfolgt analog Beispiel 9, jedoch unter Verwendung von 25 mg Wirkstoff und 125 mg POPC zur Herstellung der ethanolischen Lösung.

**Beispiel 12: Oral verabreichbare Dispersion 4**

Die Herstellung erfolgt analog Beispiel 9, jedoch unter Verwendung von 50 mg Wirkstoff und 50 mg POPC zur Herstellung der ethanolischen Lösung.

**Beispiel 13: Oral verabreichbare Dispersion 5**

Die Herstellung erfolgt analog einem der Beispiele 9 bis 12, jedoch unter Verwendung von Wirkstoff und Phosphatidylcholin aus Soja oder Phosphatidylcholin aus Eigelb (70 - 100 % rein) anstelle von POPC zur Herstellung der ethanolischen Lösung. Gewünschtenfalls wird ein Antioxidans, wie Ascorbinsäure, in einer Konzentration von 5 mg/ml zugegeben.

**Patentansprüche**

1. Verwendung von Hemmstoffen von HIV-Aspartatproteasen, oder von Salzen davon, sofern salzbildende Gruppen vorliegen, oder an Hydroxygruppen veresterten Derivaten davon oder deren Salzen, sofern salzbildende Gruppen vorliegen, zur Herstellung von pharmazeutischen Präparaten zur Anwendung zur Behandlung von Tumoren in Warmblütern.

2. Verwendung von Hemmstoffen von HIV-Aspartatproteasen der Formel I,

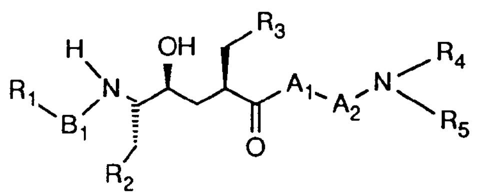

(I),

worin $R_1$ Wasserstoff, Niederalkoxycarbonyl, Heterocyclylcarbonyl, Benzyloxycarbonyl, welches unsubstituiert oder durch bis zu drei unabhängig voneinander aus Fluor, Haloniederalkyl, Niederalkanoyl, Sulfo, Niederalkylsulfonyl und Cyano ausgewählte Reste substituiert ist, Heterocyclyloxycarbonyl, worin Heterocyclyl über ein Kohlenstoffatom gebunden ist, einen der genannten Carbonylreste, worin die bindende Carbonylgruppe durch eine Thiocarbonylgruppe ersetzt ist, Heterocyclylsulfonyl, Niederalkylsulfonyl oder N-(Heterocyclylniederalkyl)-N-niederalkyl-aminocarbonyl bedeutet, $B_1$ eine Bindung oder ein bivalentes Radikal einer α-Aminosäure bedeutet, welches N-terminal mit $R_1$ und C-terminal mit der Aminogruppe am $R_2$-$CH_2$- tragenden Kohlenstoffatom verbunden ist, $R_2$ und $R_3$ unabhängig voneinander Phenyl oder Cyclohexyl, wobei diese Reste unsubstituiert oder durch ein bis drei unabhängig aus Hydroxy, Niederalkoxy, Halo, Haloniederalkyl, Sulfo, Niederalkylsulfonyl, Cyano und Nitro ausgewählte Reste substituiert sind, bedeuten, $A_1$ eine Bindung zwischen -C=O und $A_2$ oder ein bivalentes Radikal einer α-Aminosäure bedeutet, welches N-terminal mit der Gruppe -C=O und C-terminal mit $A_2$ verbunden ist, $A_2$ ein bivalentes Radikal einer α-Aminosäure bedeutet, welches N-terminal mit $A_1$ und C-terminal mit der Gruppe $NR_4R_5$ verbunden ist, oder $A_1$ und $A_2$ zusammen ein bivalentes Radikal eines Dipeptides bilden, dessen zentrale Amidbindung reduziert ist und das N-terminal mit der Gruppe -C=O und C-terminal mit der Gruppe $NR_4R_5$ verbunden ist, und $R_4$ und $R_5$ gemeinsam mit dem bindenden Stickstoffatom unsubstituiertes oder substituiertes Thiomorpholino oder Morpholino bedeuten, oder Salzen dieser Verbindungen, sofern salzbildende Gruppen vorliegen, oder hydroxygeschützten Derivaten dieser Verbindungen oder deren Salzen zur Herstellung von pharmazeutischen Präparaten zur Anwendung zur Behandlung von Tumoren in Warmblütern.

3. Verwendung nach Anspruch 2 von einer Verbindung der Formel I, oder von pharmazeutisch verwendbaren Salzen dieser Verbindungen, sofern salzbildende Gruppen vorliegen, worin $R_1$ Wasserstoff, tert-Butoxycarbonyl, Isobutyloxycarbonyl, Pyridin-3-carbonyl, Morpholinocarbonyl, 3-Benzofuranoyl, 1,2,3,4-Tetrahydro-isochinolin-3-carbonyl, mit bis zu drei unabhängig voneinander aus Fluor, Haloniederalkyl, Niederalkanoyl, Sulfo, Niederalkylsulfonyl und Cyano ausgewählten Resten substituiertes Benzyloxycarbonyl oder Heterocyclyloxycarbonyl bedeutet, worin Heterocyclyl über ein Kohlenstoffatom gebunden ist und ausgewählt ist aus Pyrrolyl, Thienyl, Imidazolyl, Pyrazolyl, Oxazolyl, Thiazolyl, Pyrazinyl, Pyrimidinyl, Indolyl, Chinolyl, Isochinolyl, Chinoxalinyl, β-Carbolinyl und einem ganz oder teilweise gesättigten Derivat dieser Reste, oder worin die Bedeutung Heterocyclyloxycarbonyl für $R_1$ fehlt, $B_1$ eine Bindung oder ein zweiwertiges Radikal einer α-Aminosäure bedeutet, welches N-terminal mit $R_1$ und C-terminal mit der Aminogruppe am $R_2$-$CH_2$- tragenden Kohlenstoffatom verbunden ist, $R_2$ und $R_3$ unabhängig voneinander Phenyl oder Cyclohexyl, wobei diese Reste unsubstituiert oder durch ein bis zwei unabhängig voneinander aus Hydroxy, Methoxy, Fluor, Sulfo, Niederalkylsulfonyl, Trifluormethyl und Cyano ausgewählte Reste substituiert sind, bedeuten, $A_1$ ein bivalentes Radikal einer hydrophoben α-Aminosäure bedeutet, welches N-terminal mit der Gruppe -C=O und C-terminal mit $A_2$ verbunden ist, $A_2$ ein bivalentes Radikal einer hydrophoben α-Aminosäure bedeutet, welches N-terminal mit $A_1$ verbunden ist und C-terminal mit dem Rest $NR_4R_5$ verbunden ist, wobei die genannten Aminosäurereste in der (D)- oder (L)-Form vorliegen; oder $A_1$ und $A_2$ zusammen ein bivalentes Radikal eines Dipeptides aus zwei hydrophoben α-Aminosäuren bilden, dessen zentrale Amidbindung reduziert ist und das N-terminal mit der Gruppe -C=O und C-terminal mit der Gruppe $NR_4R_5$ verbunden ist, und $R_4$ und $R_5$ gemeinsam mit dem bindenden Stickstoffatom

Thiomorpholino oder Morpholino bedeuten; wobei die Hydroxygruppe in Verbindungen der Formel I an dem Kohlenstoffatom, welches dem Kohlenstoffatom benachbart ist, das den Rest $R_2$-$CH_2$- trägt, frei oder in durch Niederalkanoyl geschützter Form vorliegt, insbesondere in freier Form; und wobei bei der Definition von $R_1$ Heterocyclyloxycarbonyl auch wegfallen kann.

4. Verwendung nach Anspruch 2 von einer Verbindung der Formel I, oder von pharmazeutisch verwendbaren Salzen dieser Verbindungen, sofern salzbildende Gruppen vorliegen, worin $R_1$ Wasserstoff, tert-Butoxycarbonyl, Isobutyloxycarbonyl, Pyridin-3-carbonyl, Morpholinocarbonyl, 3-Benzofuranoyl, 1,2,3,4-Tetrahydro-isochinolin-3-carbonyl, Morpholinosulfonyl oder N-(2-Pyridylmethyl)-N-methyl-aminocarbonyl bedeutet, $B_1$ eine Bindung oder ein bivalentes Radikal der $\alpha$-Aminosäure Valin bedeutet, welches N-terminal mit $R_1$ und C-terminal mit der Aminogruppe am $R_2$-$CH_2$- tragenden Kohlenstoffatom verbunden ist, $R_2$ und $R_3$ unabhängig voneinander Phenyl oder Cyclohexyl bedeuten, wobei diese Reste unsubstituiert oder durch ein bis zwei unabhängig voneinander aus Hydroxy, Methoxy, Fluor, Trifluormethyl und Cyano ausgewählte Reste substituiert sind, $A_1$ und $A_2$ zusammen ein bivalentes Radikal eines Dipeptides der Formel Val-Phe, Ile-Phe, Val-Cha, Ile-Cha, Ile-Gly, Val-Val, Val-Gly, Val-(p-F-Phe), Val-Tyr, Val-(p-$CH_3$O-Phe), Gly-(p-F-Phe) oder eines Derivates hiervon mit reduzierter zentraler Amidbindung der Formel Val(red)-Phe bilden, das N-terminal mit der Gruppe -C=O und C-terminal mit der Gruppe $NR_4R_5$ verbunden ist, und $R_4$ und $R_5$ gemeinsam mit dem bindenden Stickstoffatom Thiomorpholino oder Morpholino, bedeuten, wobei die die Hydroxygruppe in Verbindungen der Formel I an dem Kohlenstoffatom, welches dem Kohlenstoffatom benachbart ist, das den Rest $R_2$-$CH_2$- trägt, frei oder in durch Acetyl geschützter Form vorliegt.

5. Verwendung nach Anspruch 2 einer Verbindung der Formel I, worin $R_1$ Niederalkoxycarbonyl, $B_1$ eine Bindung, $R_2$ und $R_3$ Phenyl, $A_1$ Valyl, $A_2$ Phenylalanyl, und $R_4$ und $R_5$ zusammen mit dem bindenden Stickstoffatom Morpholino bedeuten.

6. Verwendung nach Anspruch 2 einer Verbindung der Formel I, worin $R_1$ Niederalkoxycarbonyl, $B_1$ eine Bindung, $R_2$ und $R_3$ Phenyl, $A_1$ Valyl, $A_2$ Phenylalanyl, und $R_4$ und $R_5$ zusammen mit dem bindenden Stickstoffatom Morpholino bedeuten.

7. Verwendung nach Anspruch 1, wobei der Hemmstoff von HIV-Aspartatprotease durch die Bezeichnung Boc-Phe[C]Phe-(L)-Val-(L)-Phe-morpholin-4-ylamid gekennzeichnet ist.

8. Verwendung nach Anspruch 2 einer Verbindung der Formel I, worin $R_1$ Niederalkoxycarbonyl, $B_1$ eine Bindung, $R_2$ Phenyl und $R_3$ 4-Methoxyphenyl, $A_1$ Valyl, $A_2$ Phenylalanyl, und $R_4$ und $R_5$ zusammen mit dem bindenden Stickstoffatom Morpholino bedeuten.

9. Verwendung nach Anspruch 2 einer Verbindung der Formel I, worin $R_1$ Niederalkoxycarbonyl, $B_1$ eine Bindung, $R_2$ Cyclohexyl und $R_3$ 4-Methoxyphenyl, $A_1$ Valyl, $A_2$ Phenylalanyl, und $R_4$ und $R_5$ zusammen mit dem bindenden Stickstoffatom Morpholino bedeuten.

10. Verwendung gemäss einem der Ansprüche 1 bis 9, dadurch gekennzeichnet, dass die zu behandelnden Tumoren solche sind, die nicht auf die Hemmung von HIV-Aspartatproteasen selbst ansprechen.

Europäisches Patentamt

# EUROPÄISCHER TEILRECHERCHENBERICHT

der nach Regel 45 des Europäischen Patent-übereinkommens für das weitere Verfahren als europäischer Recherchenbericht gilt

Nummer der Anmeldung
EP 94 81 0274

## EINSCHLÄGIGE DOKUMENTE

| Kategorie | Kennzeichnung des Dokuments mit Angabe, soweit erforderlich der maßgeblichen Teile | Betrifft Anspruch | KLASSIFIKATION DER ANMELDUNG (Int.Cl.5) |
|---|---|---|---|
| P,X | WO-A-94 05285 (THE REGENTS OF THE UNIVERSITY OF CALIFORNIA) 17. März 1994<br>* Zusammenfassung *<br>* Seite 8, Zeile 9 - Seite 9, Zeile 17; Beispiel 7 *<br>--- | 1,10 | A61K37/64 |
| X | CHEMICAL ABSTRACTS, vol. 115, no. 8, 26. August 1991, Columbus, Ohio, US; abstract no. 78928, 'PEPTIDES HAVING PARTIAL SEQUENCE OF HUMAN IMMUNODEFICIENCY VIRUS PROTEIN gp120, AND THEIR USE AS PROTEASE INHIBITORS'<br>* Zusammenfassung *<br>& JP-A-3 002 195 (NITTO DENKO CORP.) 8. Januar 1991<br>--- | 1,10 | |
| D,A | EP-A-0 532 466 (CIBA-GEIGY) 17. März 1993<br>* Zusammenfassung; Ansprüche; Beispiele *<br>---<br>-/-- | 1-10 | |
| | | | **RECHERCHIERTE SACHGEBIETE (Int.Cl.5)** |
| | | | A61K |

## UNVOLLSTÄNDIGE RECHERCHE

Nach Auffassung der Recherchenabteilung entspricht die vorliegende europäische Patentanmeldung den Vorschriften des Europäischen patentübereinkommens so wenig, daß es nicht möglich ist, auf der Grundlage einiger Patentansprüche sinnvolle Ermittlungen über den Stand der Technik durchzuführen.
Vollständig recherchierte Patentansprüche:
Unvollständig recherchierte Patentansprüche:
Nicht recherchierte Patentansprüche:
Grund für die Beschränkung der Recherche:

Siehe Ergänzungsblatt C

| Recherchenort | Abschlußdatum der Recherche | Prüfer |
|---|---|---|
| DEN HAAG | 10. August 1994 | Hoff, P |

**KATEGORIE DER GENANNTEN DOKUMENTEN**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P04C09)

Europäisches Patentamt

# EUROPÄISCHER TEILRECHERCHENBERICHT

Nummer der Anmeldung
EP 94 81 0274

| EINSCHLÄGIGE DOKUMENTE | | | KLASSIFIKATION DER ANMELDUNG (Int.Cl.5) |
|---|---|---|---|
| Kategorie | Kennzeichnung des Dokuments mit Angabe, soweit erforderlich der maßgeblichen Teile | Betrifft Anspruch | |
| A | MEDICAL HYPOTHESES,<br>Bd.37, Nr.3, 1992<br>Seiten 137 - 150<br>R.L. SHOEMAN ET AL. 'POTENTIAL ROLE OF THE VIRAL PROTEASE IN HUMAN IMMUNODEFICIENCY VIRUS TYPE 1 ASSOCIATED PATHOGENESIS'<br>* Zusammenfassung *<br>* Seite 145 *<br>--- | 1-10 | |
| A | WO-A-92 21373 (LOGOTHETOU-RELLA) 10. Dezember 1992<br>* Zusammenfassung; Ansprüche *<br>----- | 1-10 | |
| | | | RECHERCHIERTE SACHGEBIETE (Int.Cl.5) |

EPO FORM 1503 03.82 (P04C12)

EP 94 81 0274
-C-

UNVOLLSTÄNDIGE RECHERCHE

Vollständig recherchierte Patentansprüche: 5-9

Unvollständig recherchierte Patentansprüche 1-4,10

Eine Verbindung ist nicht ausreichend charakterisiert durch ihr pharmakologisches Profil sowie als "Hemmstoffen von HIV-Aspartatproteasen..."

Wegen der grossen Zahl der Verbindungen, die die Formel in Ansprüche 2-4 theoretisch definiert, musste die Recherche aus ökonomischen Gründen eingeschränkt werden.

Die Recherche beschränkte sich auf den erfinderischen Teil der Verbindungen und auf die in den Beispielen beschriebenen Substanzen.
(Art. 84, Direktive...Teil B, Kap. II.7, letzter Satz und Kap. III, 3.7).